# EUROPEAN PATENT SPECIFICATION

(11) **EP 1 859 804 B1**
(45) Date of publication and mention of the grant of the patent: **07.12.2016**
(21) Application number: 06713930.3
(22) Date of filing: 10.02.2006
(51) Int. Cl.: A61K 35/76, A61K 31/337, A61K 45/06, A61K 31/4745, A61K 35/761, A61K 38/17, A61K 48/00, A61K 31/475

(54) **ANTICANCER AGENT COMBINATION THERAPY**
MITTEL GEGEN KREBS FÜR EINE KOMBINATIONSTHERAPIE
AGENT ANTICANCEREUX POUR LA THERAPIE DE COMBINAISON

(30) Priority: 10.02.2005 JP 2005034773; 13.10.2005 JP 2005299300
(43) Date of publication of application: 28.11.2007
(73) Proprietor: Oncolys BioPharma, Inc., Tokyo 106-0032 (JP)
(72) Inventor: FUJIWARA, Toshiyoshi, -shi, Okayama, 7008558 (JP); TANAKA, Noriaki, -shi, Okayama, 7008558 (JP); KYO, Satoru c/o Kanazawa University, Ishikawa 920-0934 (JP)
(74) Representative: Woods, Geoffrey Corlett
(86) International application number: PCT/JP2006/302789
(87) International publication number: WO 2006/085689

(56) References cited:
- EP-B1- 1 763 369
- EP-B1- 1 859 793
- EP-B1- 1 891 973
- EP-B1- 1 891 974
- EP-B1- 1 905 451
- EP-B1- 1 949 902
- WO-A1-03/025190
- KAWASHIMA T. ET AL.: 'Telomerase-Specific Replication-Selective Virotherapy for Human Cancer' CLINICAL CANCER RESEARCH vol. 10, 2004, pages 285 - 292, XP002993315
- ZHANG J. ET AL.: 'Identification of Human Uroplakin II Promoter and Its Use in the Construction of CG8840, a Urothelium-specific Adenovirus Variant That Eliminates Established Bladder Tumors in Combination with Docetaxel' CANCER RESEARCH vol. 62, 2002, pages 3743 - 3750, XP002973938
- SWISHER S.G. ET AL.: 'Induction of p53-regulated Genes and Tumor Regression in Lung Cancer Patients after Intratumoral Delivery of Adenoviral p53 (INGN 201) and Radiation Therapy' CLINICAL CANCER RESEARCH vol. 9, 2003, pages 93 - 101, XP003002253
- GOLDSMITH M.E. ET AL.: 'The Histone Deacetylase Inhibitor FK228 Preferentially Enhances Adenovirus Transgene Expression in Malignant Cells' CLINICAL CANCER RESEARCH vol. 9, 2003, pages 5394 - 5401, XP003002254
- KITAZONO M. ET AL.: 'Enhanced Adenovirus Transgene Expression in Malignant Cells Treated with the Histone Deacetylase Inhibitor FR901228' CANCER RESEARCH vol. 61, 2001, pages 6328 - 6330, XP002957240
- PONG R.-C. ET AL.: 'Epigenetic Regulation of Coxsackie and Adenovirus Receptor (CAR) Gene Promoter in Urogenital Cancer Cells' CANCER RESEARCH vol. 63, 2003, pages 8680 - 8686, XP003002255
- WATANABE T. ET AL.: 'Histone deacetylase inhibitor FR901228 enhances the antitumor effect of telomerase-specific replication-selective adenoviral agent OBP-301 in human lung cancer cells' EXPERIMENTAL CELL RESEARCH vol. 312, 2006, pages 256 - 265, XP005207445
- LIU D ET AL: "Preclinical evaluation of synergistic effect of telomerase-specific oncolytic virotherapy and gemcitabine for human lung cancer", MOLECULAR CANCER THERAPEUTICS 20090401 AMERICAN ASSOCIATION FOR CANCER RESEARCH INC. USA, vol. 8, no. 4, 1 April 2009 (2009-04-01), pages 980-987, ISSN: 1535-7163
- JINNO HIDETO ET AL: "Glucuronidation of 7-ethyl-10-hydroxycamptothecin (SN-38), an active metabolite of irinotecan (CPT-11), by human UGT1A1 variants, G71R, P229Q, and Y486D.", DRUG METABOLISM AND DISPOSITION, vol. 31, no. 1, January 2003 (2003-01), pages 108-113, ISSN: 0090-9556

### SEQUENCE LISTING

<110> Oncolys BioPharma, Inc.
<120> Anticancer agent to be combined with Telomelysin
<130> G07-0042EP
<140> PCT/JP2006/302789
   <141> 2006-02-10
<150> JP2005-34773
   <151> 2005-02-10
<150> JP2005-299300
   <151> 2005-10-13
<160> 10
<170> PatentIn version 3.3
<210> 1
   <211> 455
   <212> DNA
   <213> Homo sapiens
<400> 1
   tggcccctcc ctcgggttac cccacagcct aggccgattc gacctctctc cgctggggcc     60
   ctcgctggcg tccctgcacc ctgggagcgc gagcggcgcg cgggcgggga agcgcggccc    120
   agacccccgg gtccgcccgg agcagctgcg ctgtcggggc caggccgggc tcccagtgga    180
   ttcgcgggca cagacgccca ggaccgcgct ccccacgtgg cggagggact ggggacccgg    240
   gcacccgtcc tgccccttca ccttccagct ccgcctcctc cgcgcggacc ccgccccgtc    300
   ccgacccctc ccgggtcccc ggcccagccc cctccgggcc ctcccagccc ctccccttcc    360
   tttccgcggc cccgccctct cctcgcggcg cgagtttcag gcagcgctgc gtcctgctgc    420
   gcacgtggga agccctggcc ccggccaccc ccgcg                                455
<210> 2
   <211> 899
   <212> DNA
   <213> Adenovirus
<400> 2
   acaccgggac tgaaaatgag acatattatc tgccacggag gtgttattac cgaagaaatg     60
   gccgccagtc ttttggacca gctgatcgaa gaggtactgg ctgataatct tccacctcct    120
   agccattttg aaccacctac ccttcacgaa ctgtatgatt tagacgtgac ggcccccgaa    180
   gatcccaacg aggaggcggt ttcgcagatt tttcccgact ctgtaatgtt ggcggtgcag    240
   gaagggattg acttactcac ttttccgccg gcgcccggtt ctccggagcc gcctcacctt    300
   tcccggcagc ccgagcagcc ggagcagaga gccttgggtc cggtttctat gccaaacctt    360
   gtaccggagg tgatcgatct tacctgccac gaggctggct ttccacccag tgacgacgag    420
   gatgaagagg gtgaggagtt tgtgttagat tatgtggagc accccgggca cggttgcagg    480
   tcttgtcatt atcaccggag gaatacgggg gacccagata ttatgtgttc gctttgctat    540
   atgaggacct gtggcatgtt tgtctacagt cctgtgtctg aacctgagcc tgagcccgag    600
   ccagaaccgg agcctgcaag acctacccgc cgtcctaaaa tggcgcctgc tatcctgaga    660
   cgcccgacat cacctgtgtc tagagaatgc aatagtagta cggatagctg tgactccggt    720
   ccttctaaca cacctcctga gatacacccg gtggtcccgc tgtgccccat taaaccagtt    780
   gccgtgagag ttggtgggcg tcgccaggct gtggaatgta tcgaggactt gcttaacgag    840
   cctgggcaac ctttggactt gagctgtaaa cgccccaggc cataaggtgt aaacctgtg     899
<210> 3
   <211> 1823
   <212> DNA
   <213> Adenovirus
<400> 3
   ctgacctcat ggaggcttgg gagtgtttgg aagatttttc tgctgtgcgt aacttgctgg     60
   aacagagctc taacagtacc tcttggtttt ggaggtttct gtggggctca tcccaggcaa    120
   agttagtctg cagaattaag gaggattaca agtgggaatt tgaagagctt ttgaaatcct    180
   gtggtgagct gtttgattct ttgaatctgg gtcaccaggc gcttttccaa gagaaggtca    240
   tcaagacttt ggatttttcc acaccggggc gcgctgcggc tgctgttgct tttttgagtt    300
   ttataaagga taaatggagc gaagaaaccc atctgagcgg ggggtacctg ctggattttc    360
   tggccatgca tctgtggaga gcggttgtga gacacaagaa tcgcctgcta ctgttgtctt     420
   ccgtccgccc ggcgataata ccgacggagg agcagcagca gcagcaggag gaagccaggc     480
   ggcggcggca ggagcagagc ccatggaacc cgagagccgg cctggaccct cgggaatgaa     540
   tgttgtacag gtggctgaac tgtatccaga actgagacgc attttgacaa ttacagagga     600
   tgggcagggg ctaaaggggg taaagaggga gcggggggct tgtgaggcta cagaggaggc     660
   taggaatcta gcttttagct taatgaccag acaccgtcct gagtgtatta cttttcaaca     720
   gatcaaggat aattgcgcta atgagcttga tctgctggcg cagaagtatt ccatagagca     780
   gctgaccact tactggctgc agccagggga tgattttgag gaggctatta gggtatatgc     840
   aaaggtggca cttaggccag attgcaagta caagatcagc aaacttgtaa atatcaggaa     900
   ttgttgctac atttctggga acggggccga ggtggagata gatacggagg atagggtggc     960
   ctttagatgt agcatgataa atatgtggcc gggggtgctt ggcatggacg gggtggttat     1020
   tatgaatgta aggtttactg gccccaattt tagcggtacg gttttcctgg ccaataccaa    1080
   ccttatccta cacggtgtaa gcttctatgg gtttaacaat acctgtgtgg aagcctggac    1140
   cgatgtaagg gttcggggct gtgcctttta ctgctgctgg aagggggtgg tgtgtcgccc    1200
   caaaagcagg gcttcaatta agaaatgcct ctttgaaagg tgtaccttgg gtatcctgtc    1260
   tgagggtaac tccagggtgc gccacaatgt ggcctccgac tgtggttgct tcatgctagt    1320
   gaaaagcgtg gctgtgatta agcataacat ggtatgtggc aactgcgagg acagggcctc    1380
   tcagatgctg acctgctcgg acggcaactg tcacctgctg aagaccattc acgtagccag    1440
   ccactctcgc aaggcctggc cagtgtttga gcataacata ctgacccgct gttccttgca    1500
   tttgggtaac aggagggggg tgttcctacc ttaccaatgc aatttgagtc acactaagat    1560
   attgcttgag cccgagagca tgtccaaggt gaacctgaac ggggtgtttg acatgaccat    1620
   gaagatctgg aaggtgctga ggtacgatga gacccgcacc aggtgcagac cctgcgagtg    1680
   tggcggtaaa catattagga accagcctgt gatgctggat gtgaccgagg agctgaggcc    1740
   cgatcacttg gtgctggcct gcacccgcgc tgagtttggc tctagcgatg aagatacaga    1800
   ttgaggtact gaaatgtgtg ggc 1823
<210> 4
   <211> 605
   <212> DNA
   <213> Adenovirus
<400> 4
   tgcatctagg gcggccaatt ccgcccctct ccctcccccc cccctaacgt tactggccga 60
   agccgcttgg aataaggccg gtgtgcgttt gtctatatgt gattttccac catattgccg 120
   tcttttggca atgtgagggc ccggaaacct ggccctgtct tcttgacgag cattcctagg 180
   ggtctttccc ctctcgccaa aggaatgcaa ggtctgttga atgtcgtgaa ggaagcagtt 240
   cctctggaag cttcttgaag acaaacaacg tctgtagcga ccctttgcag gcagcggaac 300
   cccccacctg gcgacaggtg cctctgcggc caaaagccac gtgtataaga tacacctgca 360
   aaggcggcac aaccccagtg ccacgttgtg agttggatag ttgtggaaag agtcaaatgg 420
   ctctcctcaa gcgtattcaa caaggggctg aaggatgccc agaaggtacc ccattgtatg 480
   ggatctgatc tggggcctcg gtgcacatgc tttacatgtg tttagtcgag gttaaaaaaa 540
   cgtctaggcc ccccgaacca cggggacgtg gttttccttt gaaaaacacg atgataagct 600
   tgcca 605
<210> 5
   <211> 20
   <212> DNA
   <213> Artificial
<220>
   <223> primer
<400> 5
   acaccgggac tgaaaatgag 20
<210> 6
   <211> 21
   <212> DNA
   <213> Artificial
<220>
   <223> primer
<400> 6
   cacaggttta caccttatgg c 21
<210> 7
   <211> 20
   <212> DNA
   <213> Artificial
<220>
   <223> primer
<400> 7
   ctgacctcat ggaggcttgg 20
<210> 8
   <211> 19
   <212> DNA
   <213> Artificial
<220>
   <223> primer
<400> 8
   gcccacacat ttcagtacc 19
<210> 9
   <211> 20
   <212> DNA
   <213> Artificial
<220>
   <223> primer
<400> 9
   cctgtgtcta gagaatgcaa 20
<210> 10
   <211> 20
   <212> DNA
   <213> Artificial
<220>
   <223> primer
<400> 10
   acagctcaag tccaaaggtt 20

## Description

### TECHNICAL FIELD

The present invention relates to a pharmaceutical composition for use in combination therapy for tumors, comprising a recombinant virus comprising a polynucleotide containing a promoter for human telomerase, an E1A gene, an IRES sequence and an E1B gene in this order, and a substance having an antitumor effect.

### BACKGROUND ART

A recombinant virus growing selectively in tumor cells has an antitumor effect by itself because the viral growth induces cell death in tumor cells. However, combined use of such a virus with a substance having an antitumor effect makes it possible to inhibit cancer cell growth and thereby to secure sufficient time for the virus to grow in cancer cells. Besides, the virus that has grown in cancer cells is destroyed by anticancer agents, which promotes viral release. As a result, rapid viral diffusion into surrounding cancer cells and remarkable antitumor effect can be expected. Further, since the mode of action of cell death caused by viruses and the mode of action of cell death caused by conventional anticancer agents are different, the possibility that respective antitumor effects when they are combined will be inhibited would be presumably low. Actually, it is reported that the antitumor effects of herpes viruses growing selectively in tumor cells have been enhanced pre-clinically and clinically when they were used in combination with conventional anticancer therapies such as with anticancer agents or radiation (Post DE, Fulci G, Chiocca EA, and Van Meir EG. "Replicative oncolytic herpes simplex viruses in combination cancer therapies", Curr Gene Ther. 2004 Mar; 4(1):41-51; and Bennett JJ, Adusumilli P, Petrowsky H, Burt BM, Roberts G, Delman KA, Zager JS, Chou TC, and Fong Y. "Up-regulation of GADD34 mediates the synergistic anticancer activity of mitomycin C and a gamma134.5 deleted oncolytic herpes virus (G207)", FASEB J. 2004 Jun; 18(9):1001-3).

Kawashima et al (2004) Clinical Cancer Research, 10, 285-292, discloses a recombinant virus having the genetic configuration hTERT promoter-E1A-IRES-E1B.

Zhang et al (2002) Cancer Research, 62, 3743-3750 and Goldsmith et al (2003) Clinical Cancer Research, 9, 5394-5401 disclose adenoviral constructs with particular promoters in combination with an anti-tumour substance.

### DISCLOSURE OF THE INVENTION

It is an object of the present invention to provide a pharmaceutical composition with still enhanced anticancer activity.

As a result of extensive and intensive researches toward the solution of the above problem, the present inventors have found that combined use of a recombinant virus comprising a polynucleotide containing a promoter for human telomerase, an E1A gene, an IRES sequence and an E1B gene in this order with a substance having an antitumor effect enhances anticancer activity. Thus, the present invention has been achieved.

The present invention relates to the following.
- A pharmaceutical composition for use in a method of treatment of tumours by combination therapy, comprising a recombinant adenovirus comprising a polynucleotide containing a promoter for human telomerase, an E1A gene, an IRES sequence and an E1B gene in this order, and a substance having an anti-tumor effect, wherein the substance having an anti-tumour effect is vinorelbine, a topoisomerase I inhibitor, INGN-201 or a substance having antimetabolic activity.

In the above-described pharmaceutical composition, as an example of the human telomerase, hTERT may be given. Examples of substances having topoisomerase I inhibitory activity include irinotecan or salts thereof. The tumor to be treated is not particularly limited. For example, lung cancer, large bowel cancer, gastric cancer, breast cancer, esophageal cancer, head and neck cancer, liver cancer, pancreatic cancer, gallbladder/bile duct cancer, prostate cancer, bladder cancer, cervical cancer, thyroid cancer, ovarian cancer, leukemia, lymphoma, sarcoma, mesenchymal tumor and so on may be enumerated.
- A recombinant adenovirus comprising a polynucleotide containing a promoter for human telomerase, an E1A gene, an IRES sequence and an E1B gene in this order for use in a method of treating tumors in combination with a substance having an anti-tumour effect selected from vinorelbine, a topoisomerase I inhibitor, INGN-201 or a substance having antimetabolic activity.
- A substance having an anti-tumour effect selected from vinorelbine, a topoisomerase I inhibitor, INGN-201 or a substance having antimetabolic activity for use in a method of treating tumors in combination with a recombinant adenovirus comprising a polynucleotide containing a promoter for human telomerase, an E1A gene, an IRES sequence and an E1B gene in this order.

The tumor to be treated is not particularly limited. For example, lung cancer, large bowel cancer, gastric cancer, breast cancer, esophageal cancer, head and neck cancer, liver cancer, pancreatic cancer, gallbladder/bile duct cancer, prostate cancer, bladder cancer, cervical cancer, thyroid cancer, ovarian cancer, leukemia, lymphoma, sarcoma, mesenchymal tumor and so on may be enumerated.
- Use of a recombinant adenovirus comprising a polynucleotide containing a promoter for human telomerase, an E1A gene, an IRES sequence and an E1B gene in this order for the manufacture of a medicament for use in a method of treating tumors in combination with a substance having an anti-tumour effect selected from vinorelbine, a topoisomerase I inhibitor, INGN-201 or a substance having antimetabolic activity.
- Use of a substance having an anti-tumour effect selected from vinorelbine, a topoisomerase I inhibitor, INGN-201 or a substance having antimetabolic activity for the manufacture of a medicament for use in a method of treating tumors in combination with a recombinant adenovirus comprising a polynucleotide containing a promoter for human telomerase, an E1A gene, an IRES sequence and an E1B gene in this order.

### BRIEF DESCRIPTION OF THE DRAWINGS

Fig. 1 shows a schematic diagram of the recombinant adenovirus (Telomelysin) of the present invention.

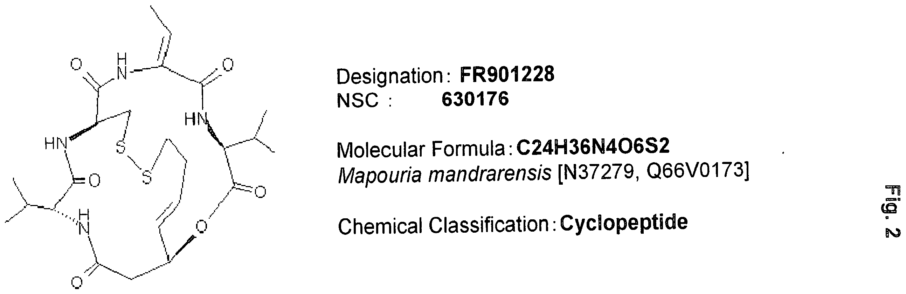

Fig. 2 shows FR901228.

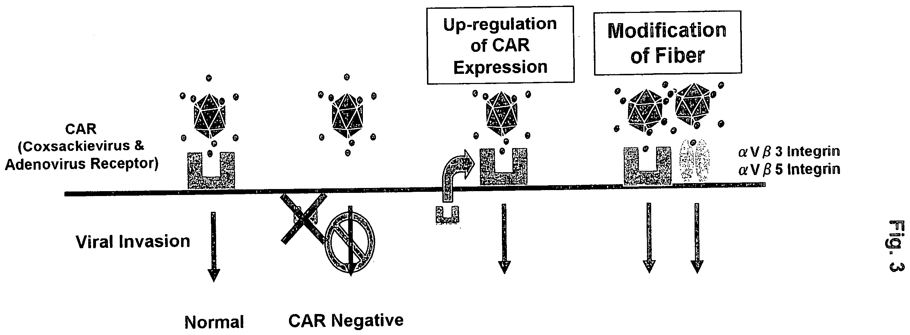

Fig. 3 is a diagram showing the effect of CAR expression on adenovirus infection.
Fig. 4 is graphs showing the results of analysis of the combined effect of Telomelysin and a microtubule inhibitor using XTT assay.
Fig. 5 is graphs showing the results of XTT assay on Telomelysin combined with a microtubule inhibitor vinorelbine or a topoisomerase I inhibitor SN-38 (7-ethyl-10-hydroxycamptothecin; CPT-11 metabolite).
Fig. 6 is graphs showing the results of analysis of the effect of an anticancer agent on the growth of the recombinant virus.
Fig. 7 is graphs showing the effects of Telomelysin and a microtubule inhibitor upon the cell cycle of tumor cells.
Fig. 8 shows the *in vivo* antitumor effect of Telomelysin and a microtubule inhibitor in H1299 human lung cancer cell.
Fig. 9 is photographs showing histological changes (HE staining) in tumor tissues and the liver after intratumoral administration of Telomelysin.
Fig. 10 is graphs showing the expression of an adenovirus receptor CAR by an HDAC inhibitor FR901228.
Fig. 11 is photographs showing the adenovirus infection efficiency by an HDAC inhibitor FR901228.
Fig. 12 is graphs showing the enhancement of OBP-401 infection efficiency by an HDAC inhibitor FR901228.
Fig. 13 is graphs showing the enhancement of the antitumor effect of Telomelysin by an HDAC inhibitor FR901228.
Fig. 14 is a graph showing measurement results of the antitumor effect of Advexin.
Fig. 15 is a graph showing the results of measurement of the antitumor effect of Telomelysin-Advexin simultaneous administration.
Fig. 16 is a graph showing the results of measurement of the antitumor effect of Telomelysin-Advexin non-simultaneous administration.
Fig. 17 is a graph showing the results of measurement of the antitumor effect of Telomelysin-Advexin non-simultaneous administration.
Fig. 18 is a graph showing the results of measurement of the antitumor effect of Telomelysin-Advexin non-simultaneous administration.

### BEST MODE FOR CARRYING OUT THE INVENTION

The present invention relates to a pharmaceutical composition for use in combination therapy for tumors, comprising a recombinant adenovirus comprising a polynucleotide containing a promoter for human telomerase, an E1A gene, an IRES sequence and an E1B gene in this order, and a substance having an antitumor effect. The pharmaceutical composition not only produces an antitumor effect superior to that produced by the recombinant virus or the antitumor agent alone, but also exerts an antitumor effect even on those tumor cells which are not affected by such substances when used alone. Hereinbelow, the present invention will be described in detail.

### 1. The Recombinant Virus

The recombinant virus means a virus in which a polynucleotide comprising a promoter for human telomerase, an E1A gene, an IRES sequence and an E1B gene in this order has been integrated into its genome. The recombinant virus is an adenovirus. Among adenoviruses, type 5 adenovirus is especially preferable because of its easiness in handling, etc.

In the recombinant virus used in the present invention, the E1A gene, the IRES sequence and the E1B gene are driven by the promoter for human telomerase. Since expression of telomerase is extremely high in tumor cells compared to normal cells, the telomerase promoter is expressed in telomerase-containing tumor cells and, as a result, the recombinant virus proliferates. Thus, the recombinant virus contained in the pharmaceutical composition does not proliferate in normal cells and proliferates only in tumor cells. This means the recombinant virus is proliferated/replicated tumor cell-specifically and telomerase-specifically As a result, viral growth causes cytotoxicity in tumor cells, by which the recombinant virus used in the present invention can kill tumor cells specifically.

The "telomerase promoter" determines the transcription initiation site for telomerase and directly regulates the frequency of transcription. Telomerase is an enzyme that maintains the length of telomeres, standing against the shortening of telomeres at the time of replication of eukaryotic chromosomes. The type of such telomerase promoter is not particularly limited, and any suitable telomerase promoter compatible with the virus to be used for the expression of a gene of interest may be used. For example, the promoter for human telomerase reverse transcriptase (hTERT) (hereinafter, referred to as "hTERT promoter") is preferable. A great number of transcription factor-binding sequences are confirmed in a 1.4 kbp region upstream of the 5' end of hTERT gene. This region is believed to be hTERT promoter. In particular, a 181 bp sequence located upstream of the translation initiation site is a core region important for the expression of the downstream gene. In the present invention, any sequence comprising this core region may be used. Preferably, an upstream sequence of approximately 378 bp containing the entire core region is used as hTERT promoter. It has been confirmed that this sequence of approximately 378 bp is equivalent to the 181 bp core region alone in gene expression efficiency The nucleotide sequence of an hTERT promoter of 455 bp is shown in SEQ ID NO: 1.

The nucleotide sequence of hTERT promoter is not limited to the sequence as shown in SEQ ID NO: 1. Nucleotide sequences of nucleotides which hybridize under stringent conditions to a DNA consisting of a nucleotide sequence complementary to the DNA consisting of SEQ ID NO: 1 and have hTERT promoter activity may also be included as a sequence for hTERT promoter. Such nucleotides may be obtained from cDNA libraries or genomic libraries by known hybridization methods such as colony hybridization, plaque hybridization, Southern blotting, etc. using the polynucleotide consisting of the nucleotide sequence of SEQ ID NO: 1 or a part thereof as a probe. cDNA libraries may be prepared according to the method described in Molecular Cloning: A Laboratory Manual 2nd ed. (Cold Spring Harbor Press (1989)). Alternatively, commercial cDNA libraries or genomic libraries may be used. In the above hybridization, examples of stringent conditions include 1xSSC to 2xSSC, 0.1% to 0.5% SDS and 42°C to 68°C. More specifically, an example may be given where prehybridization is performed at 60-68°C for more than 30 minutes, and then washing is performed in 2xSSC, 0.1% SDS at room temperature for 5-15 minutes 4 to 6 times. For detailed procedures of hybridization, see, for example, Molecular Cloning: A Laboratory Manual 2nd ed. (Cold Spring Harbor Press (1989), in particular, Section 9.47-9.58). Further, nucleotide sequences of nucleotides which have at least 50% or more (e.g., 60% or more, 70% or more, 80% or more, 90% or more, 95% or more, 98% or more, or 99% or more) homology to the nucleotide sequence as shown in SEQ ID NO: 1 and have hTERT promoter activity may also be used as a sequence for hTERT promoter.

The reason why an E1A gene, an IRES sequence and an E1B gene are located in this order in the present invention is that insertion of the IRES sequence between the E1A gene and E1B gene will results in higher replication ability of the virus when a host cell has been infected with it. E1A gene and E1B gene are genes included in E1 gene. This is one of early genes of viruses, which have early (E) genes and late (L) genes involved in their DNA replication, and encodes a protein involved in the regulation of transcription of viral genome. E1A protein encoded by E1A gene activates the transcription of a group of genes (E1B, E2, E4, etc.) necessary for the production of infectious virus. E1B protein encoded by E1B gene assists the accumulation of late gene (L gene) mRNA in the cytoplasm of the infected host cell to thereby inhibit the protein synthesis in the host cell. Thus, E1B protein promotes viral replication. The nucleotide sequences of E1A gene and E1B gene are shown in SEQ ID NO: 2 and SEQ ID NO: 3, respectively.

E1A and E1B may comprise, other than those nucleotide sequences shown in SEQ ID NO: 2 and SEQ ID NO: 3, respectively, nucleotide sequences which hybridize under stringent conditions to a DNA consisting of a nucleotide sequence complementary to the DNA consisting of SEQ ID NO: 2 or SEQ ID NO: 3 and encode a protein having E1A or E1B activity Such nucleotide sequences may be obtained from cDNA libraries or genomic libraries by known hybridization methods such as colony hybridization, plaque hybridization, Southern blotting, etc. using the polynucleotide, or a part thereof, consisting of the nucleotide sequence of SEQ ID NO: 2 or SEQ ID NO: 3 as a probe. cDNA libraries may be prepared according to the method described in Molecular Cloning: A Laboratory Manual 2nd ed. (Cold Spring Harbor Press (1989)). Alternatively, commercial cDNA libraries or genomic libraries may be used. In the above hybridization, examples of stringent conditions include 1xSSC to 2xSSC, 0.1% to 0.5% SDS and 42°C to 68°C. More specifically, an example may be given where prehybridization is performed at 60-68°C for more than 30 minutes, and then washing is performed in 2xSSC, 0.1% SDS at room temperature for 5-15 minutes 4 to 6 times. For detailed procedures of hybridization, see, for example, Molecular Cloning: A Laboratory Manual 2nd ed. (Cold Spring Harbor Press (1989), in particular, Section 9.47-9.58). Further, nucleotide sequences of nucleotides which have at least 50% or more (e.g., 60% or more, 70% or more, 80% or more, 90% or more, 95% or more, 98% or more, or 99% or more) homology to the nucleotide sequence as shown in SEQ ID NO: 2 or 3 and have E1A activity (when having the above-described homology to SEQ ID NO: 2) or have E1B activity (when having the above-described homology to SEQ ID NO: 3) may also be used.

IRES (Internal Ribosome Entry Site) is a protein synthesis initiation signal specific to picornavirus. It is believed that this site serves as a ribosome-binding site because it has a complementary sequence to the 3' terminal sequence of 18S ribosomal RNA. It is known that mRNA derived from a virus which belongs to picornaviridae is translated via this sequence. Translation efficiency from IRES sequence is high. Even from the middle of mRNA, protein synthesis is performed in a cap structure non-dependent manner. Therefore, in the virus of the present invention, both E1A gene and E1B gene (which is located downstream of the IRES sequence) are translated independently by a human telomerase promoter. By using IRES, the control of expression by a telomerase promoter is exerted on E1A gene and E1B gene independently. Therefore, compared to cases where either E1A gene or E1B gene is controlled by a telomerase promoter, viral replication can be more strictly limited to those cells having telomerase activity An IRES sequence is shown in SEQ ID NO: 4.

IRES may comprise, other than the nucleotide sequence as shown in SEQ ID NO: 4, nucleotide sequences which hybridize under stringent conditions to a DNA consisting of a nucleotide sequence complementary to the DNA consisting of SEQ ID NO: 4 and encode a protein having IRES activity. Such nucleotide sequences may be obtained from cDNA libraries or genomic libraries by known hybridization methods such as colony hybridization, plaque hybridization, Southern blotting, etc. using the polynucleotide, or a part thereof, consisting of the nucleotide sequence of SEQ ID NO: 4 as a probe. cDNA libraries may be prepared according to the method described in Molecular Cloning: A Laboratory Manual 2nd ed. (Cold Spring Harbor Press (1989)). Alternatively, commercial cDNA libraries or genomic libraries may be used. In the above hybridization, examples of stringent conditions include 1xSSC to 2xSSC, 0.1% to 0.5% SDS and 42°C to 68°C. More specifically, an example may be given where prehybridization is performed at 60-68°C for more than 30 minutes, and then washing is performed in 2xSSC, 0.1% SDS at room temperature for 5-15 minutes 4 to 6 times. For detailed procedures of hybridization, see, for example, Molecular Cloning: A Laboratory Manual 2nd ed. (Cold Spring Harbor Press (1989), in particular, Section 9.47-9.58). Further, nucleotide sequences of nucleotides which have at least 50% or more (e.g., 60% or more, 70% or more, 80% or more, 90% or more, 95% or more, 98% or more, or 99% or more) homology to the nucleotide sequence as shown in SEQ ID NO: 4 and have IRES activity may also be used.

In the present invention, a promoter for human telomerase is located upstream of the E1 gene because such a promoter is capable of promoting viral proliferation in cells having telomerase activity.

The genes contained in the recombinant virus may be obtained by conventional genetic engineering techniques. For example, as a genetic engineering technique, a method of nucleic acid synthesis with a commonly used DNA synthesizer may be used. Alternatively, after a genetic sequence to be used as a template is isolated or synthesized, primers specific to each gene may be designed and the genetic sequence may be amplified with a PCR apparatus (PCR method; Current Protocols in Molecular Biology, John Wiley & Sons (1987), Section 6.1-6.4); or a gene amplification method using a cloning vector may be used. One of ordinary skill in the art can readily carry out the above methods according to, for example, Molecular Cloning 2nd Ed., Cold Spring Harbor Laboratory Press (1989). Purification of resultant PCR products may be performed by known methods such as a method using ethidium bromide, a method using SYBR Green I (Molecular Probes), a method with GENECLEAN (Funakoshi), QIAGEN (QIAGEN), etc. using agarose gel, a method using DEAE-cellulose filter, freeze & squeeze method or a method using a dialysis tube. When agarose gel is used, PCR products are electrophoresed on agarose gel and resultant DNA fragments are cut out from the gel and purified. If necessary, it is possible to confirm by conventional sequencing methods that an expected gene has been obtained. For example, dideoxynucleotide chain termination method (Sanger et al. (1977) Proc. Natl. Acad. Sci. USA 74: 5463) or the like may be used for this purpose. Alternatively, it is also possible to analyze the sequence with an appropriate DNA sequencer (e.g., ABI PRISM; Applied Biosystems).

Subsequently, individual genes thus obtained are ligated in a specific order. First, above genes are digested with known restriction enzymes, and the resultant DNA fragments are inserted into a known vector according to a known method for ligation. Specific examples of known vectors include pIRES vector which comprises the IRES (internal ribosome entry site in mRNA) of encephalomyocarditis virus (ECMV) and is capable of translating two open reading frames (ORFs) from one mRNA; *Escherichia coli*-derived plasmids (such as pCR4, pCR2, pCR2.1, pBR322, pBR325, pUC12 and pUC13); Bacillus subtilis-derived plasmids (such as pUB110, pTP5 and pC194); yeast-derived plasmids (such as pSH19 and pSH15); bacteriophages such as À phage; animal viruses such as retrovirus, vaccinia virus and baculovirus; pA1-11, pXT1, pRc/CMV, pRc/RSV, pcDNAI/Neo and so forth. In the present invention, use of pIRES vector is preferable. With this vector, it is possible to prepare a recombinant gene comprising "a promoter for human telomerase, an E1A gene, an IRES sequence and an E1B gene" in this order by inserting necessary genes into the multi-cloning site in this order. DNA ligase may be used for ligation of DNAs. For the integration of the thus prepared recombinant gene into a virus, methods such as electroporation, the liposome method, the spheroplast method, the lithium acetate method or the like may be used.

Hereinbelow, an example where hTERT is used as human telomerase will be described specifically.

E1A gene and E1B gene may be amplified from E1 gene-expressing cells (such as 293 cells) by carrying out RT-PCR and/or DNA-PCR using primers such as E1A-S, E1A-AS, E1B-S and E1B-AS. If necessary, their sequences are confirmed using a known method such as TA cloning. Then, DNA fragments of E1A and E1B may be cut out using known restriction enzymes.

Subsequently, a gene consisting of hTERT-E1A-IRES-E1B to be used in the present invention may be prepared by inserting individual genes into a multi-cloning site or the like of a known vector (such as pIRES vector) to give the following order: "E1A-IRES-E1B". Then, the sequence of hTERT promoter cut out with restriction enzymes MluI, BglIII, etc. may be inserted upstream of E1A.

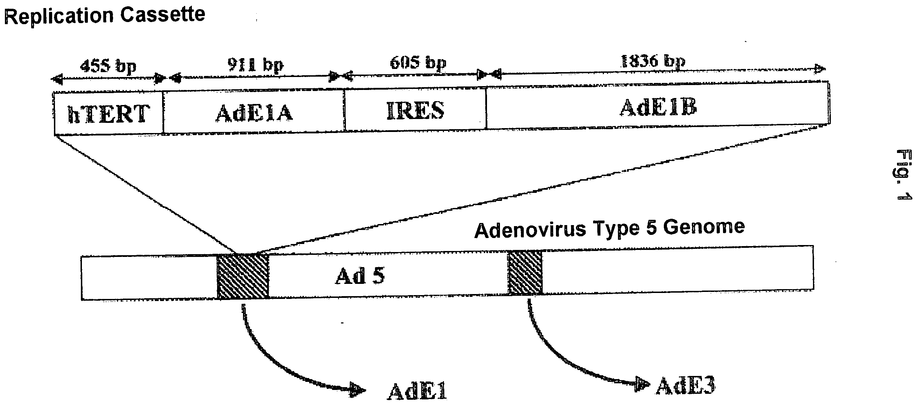

If necessary, it is also possible to remove a cytomegalovirus (CMV) promoter from a known vector such as pShuttle with appropriate restriction enzymes such as MfeI and NheI, and to insert into that site a sequence cut out from phTERT-E1A-IRES-E1B with restriction enzymes NheI and NotI. The adenovirus to be used in the present invention in which the replication cassette consisting of hTERT-E1A-IRES-E1B (Fig. 1) has been integrated is particularly designated "Telomelysin". By expressing E1 gene necessary for proliferation of adenovirus under the control of hTERT promoter, it is possible to allow the virus to proliferate in a cancer cell-specific manner.

For infecting cells with a recombinant virus, the following method may be used, for example. First, cells such as human large bowel cancer cell SW620, human lung cancer cells A549 and H1299 are plated in culture plates containing an appropriate culture broth and cultured in the presence of CO₂ gas at 37°C. As the culture broth, one which is conventionally used for culturing animal cells may be used, e.g. DMEM, MEM, or RPMI-1640. If necessary, serum, antibiotics, vitamins, or the like may be added thereto. By inoculating a specific amount of the recombinant virus of the present invention (0.1-10 MOI (multiplicity of infection), preferably 0.1-1 MOI), the cultured cells are infected. MOI means a ratio between the viral quantity (infective unit) and the cell count when a specific amount of cultured cells are infected with a specific amount of viral particles. MOI is used as an indicator for viral infection.

In order to confirm viral replication, cells infected with virus are collected, and DNA is extracted therefrom. Then, the DNA is subjected to real-time PCR using primers targeting an appropriate gene contained in the virus. Thus, quantitative analysis is possible.

### 2. Substances Having Antitumor Effect

The "substance having an antitumor effect" refers to a substance having an effect of inhibiting the growth or proliferation of tumor cells (cancer cells) which form tumor masses as a result of repeated cell division and hyperproliferation caused by disorder of the regulatory mechanism of the body. Substances which inhibit the proliferation of cancer cells by inhibiting the nucleic acid synthesis of cancer cells or by inhibiting the metabolism of cancer cells are also included in the substance having an antitumor effect. Such substances include substances having antimetabolic activity that antagonizes enzymes in metabolic processes to thereby inhibit cell synthesis; substances having microtubule inhibitory activity (vinorelbine) that acts on microtubules and exhibits an antitumor effect; and substances having topoisomerase inhibitory activity that inhibits topoisomerase. Topoisomerase is an enzyme that catalyzes a reaction of changing the linking number of DNA by transiently cutting one or both strands of DNA. Specific examples of each type of the above-described substances are given below.
(1) Substances having antimetabolic activity: methotrexate (folates), mercaptopurine (purines), cytarabine (pyrimidines), fluorouracil, tegafur, carmofur, etc.
(2) Substances having microtubule inhibitory activity: vinorelbine.
(3) Substances having topoisomerase inhibitory activity: irinotecan (topoisomerase I inhibitor).

In the present invention, salts of the above-described substances having an antitumor effect may also be used. The "salts" suitable for the present invention are not particularly limited. For example, various inorganic acid addition salts such as hydrochloride, hydrobromide, sulfate, phosphate, nitrate, pyrosulfate, metaphosphate, and hydroiodide; various organic acid addition salts such as acetate, propionate, succinate, glycolate, lactate, malate, oxalate, tartarate, citate, maleate, fumarate, methanesulfonate, benzenesulfonate, p-toluenesulfonate, ascorbate and benzoate; and salts formed with various amino acids such as aspartate and glutamate; may be enumerated. Further, when the above-described substance has phenolic hydroxyl group or carboxyl group, the substance may also be used in the form of an alkali metal salt such as sodium salt or potassium salt.

Further, in the present invention, hydrates or non-hydrates of the above-described substance may be used. In non-hydrates, alcohol (e.g., methanol, ethanol, or n-propanol), dimethyl formamide or the like may be used.

The above-described substance may be obtained by known chemical synthesis or as a commercial product.

Among the above-described anticancer agents, especially preferable for the present invention is vinorelbine (which is a substance having microtubule inhibitory activity) and irinotecan (which is a substance having topoisomerase inhibitory activity). However, anticancer agents which may be used in the present invention are not limited to these substances.

Vinorelbine is classified into the group of plant (vinca) alkaloids in the above-described microtubule inhibitors and applied to the treatment of non-small-cell lung cancer. Besides, a clinical trial of vinorelbine as an injection drug for breast cancer is now proceeding. Vinorelbine is low in toxicity, acts tubulin-selectively to inhibit the polymerization, and interferes with cell division to thereby induce cell death.

Irinotecan is classified into the above-described substances having topoisomerase I inhibitory activity, and is applied to the treatment of various cancers such as large bowel cancer, several malignant lymphomas, small-cell lung cancer, non-small-cell lung cancer, cervical cancer, ovarian cancer, gastric cancer (inoperable or recurrent), colon/rectal cancer (inoperable or recurrent), breast cancer (inoperable or recurrent), squamous cell carcinoma, and malignant lymphoma (non-Hodgkin's lymphoma). Irinotecan is camptothecin (vinca alkaloid) extracted from *Camptotheca acuminata*, a plant whose place of origin is China. This substance interferes the development of elements necessary for cell division to thereby inhibit the growth of cancer cells.

Further, INGN-201 (Advexin; Introgen Therapeutics), which is a substance used in gene therapy is also effective as a substance to be included in the pharmaceutical composition. Gene therapy is a tumor inhibitory therapy, i.e., a method of treating tumors by expressing a protein having immunopotentiation activity or angiogenesis inhibitory effect in the body transiently or constitutively. INGN-201 is a gene therapeutic drug in which a tumor suppressor gene p53 has been integrated into an adenovirus. This adenovirus is replication-incompetent and expresses p53 protein. INGN-201 exerts antitumor effect synergistically with Telomelysin in certain types of tumor cells.

Unlike Telomelysin, INGN-201 exerts its antitumor effect by inducing apoptotic cell death in cancer cells. When INGN-201 and Telomelysin infect cancer cells simultaneously, the replication-incompetent INGN-201 becomes replicable by using the E1 protein produced by Telomelysin. p53 protein is produced in a large quantity by this INGN-201, inducing apoptosis. At the same time, cell death caused by Telomelysin also occurs. Therefore, a very potent cytocidal effect is expected.

The target tumor (cancer) cell of the pharmaceutical composition is not particularly limited. Any type of tumor cell may be used. For example, the pharmaceutical composition is effective for solid tumors in the head and neck, stomach, large bowel, lung, liver, prostate, pancreas, esophagus, bladder, gallbladder/bile duct, breast, uterus, thyroid, ovary, etc.; or leukemia, lymphoma, sarcoma, mesenchymal tumor, or the like. Most of tumor cells derived from human tissues show increased telomerase activity. The pharmaceutical composition is capable of acting generally on vigorously proliferating tumor cells by means of such telomerase activity. The pharmaceutical composition is especially effective for organs such as large bowel, lung, stomach, esophagus, liver, prostate, head and neck, breast, etc. on which the substance having an antitumor effect (anticancer agent) acts.

As described above, since telomerase expression is extremely high in tumor cells compared to normal cells, the recombinant virus contained in the pharmaceutical composition does not proliferate in normal cells but in tumor cells alone. As a result, the virus is capable of killing tumor cells specifically.

### 3. Pharmaceutical Composition

The pharmaceutical composition is characterized by the combined use of a recombinant virus comprising a polynucleotide containing a promoter for human telomerase, an E1A gene, an IRES sequence and an E1B gene in this order with a substance having an antitumor effect.

This has been achieved based on the fact that the mode of action of Telomelysin recombinant virus inducing cell death in tumor cells is different from the mode of action of conventional anticancer agents inducing apoptosis; and the nature of Telomelysin that even when tumor cells have been treated with a substance having an antitumor effect, the recombinant virus is capable of proliferation/replication in the tumor cells without being affected by the substance.

The pharmaceutical composition may be applied to the diseased site as it is. Alternatively, the agent may be introduced into the living body (target cell or organ) by any known method, e.g. intravenous, intramuscular, intra-abdominal or subcutaneous injection; inhalation through the nasal cavity, oral cavity or lung; oral administration; intravascular administration using catheter or the like. Further, the recombinant virus and the substance having an antitumor effect contained in the pharmaceutical composition may be administered to the living body either simultaneously or non-simultaneously, i.e., one may be administered first and the other may be administered after a while.

The pharmaceutical composition may be treated, for example, by the method such as freezing to enable easy handling and then used as it is, or mixed with known pharmaceutically acceptable carriers such as excipients, fillers, binders, lubricants; or known additives (including such as buffers, isotonic agents, chelating agents, coloring agents, preservatives, fragrances, flavoring agents, and sweetening agents). When the recombinant virus and the substance having an antitumor effect contained in the pharmaceutical composition are administered separately, the above-mentioned substances may be added to each of them.

The pharmaceutical composition may be administered orally or parenterally depending on the form of the agent, e.g. oral administration agents such as tablets, capsules, powders, granules, pills, liquids, syrups, etc. and parenteral administration agents such as injections, external medicines, suppositories, eye drops, etc. Preferably, local injection into muscle or abdominal cavity, or intravenous injection may be used.

Dose levels are selected appropriately depending on the kind of active ingredient, the administration route, the target of administration, and the age, body weight, sex, symptoms and other conditions of the patient. The virus contained in the pharmaceutical composition may be administered at a daily dose of about 10⁶-10¹¹ PFU (plaque forming units), preferably about 10⁹-10¹¹ PFU. This amount may be administered once a day, or may be divided into several portions and administered at several times a day.

When the virus is administered, it is also possible to use a known immunosuppressant or the like to suppress the immunity of the living body to thereby facilitate the viral infection. Further, the virus may be used jointly with at least one anticancer agent selected from the group consisting of known anticancer agents and radiation.

The possibility that the pharmaceutical composition will produce side effects is believed to be extremely low because of the reasons described below. Thus, the pharmaceutical composition can be said a very safe preparation.
(1) There is little telomerase activity in normal somatic cells, and the virus is hard to be infectious in suspension cells such as hematopoietic cells.
(2) Since the virus has replication ability, it is possible to use this virus at a lower concentration than that of non-replication competent viruses used in conventional gene therapy.
(3) Even when the virus has been administered in excess, antiviral action works via ordinary immune reaction in the living body.

Dose levels of the substance having an antitumor effect are also selected appropriately depending on the kind of active ingredient, the administration route, the target of administration, and the age, body weight, sex, symptoms and other conditions of the patient. When vinorelbine is used, it may be injected intravenously and slowly at 2.0-2.5 mg/m² per administration at intervals of one week.

When irinotecan is used, usually, it is administered in the form of, for example, irinotecan hydrochloride to an adult patient by intravenous infusion once a day at 150 mg/m² at intervals of 2 weeks 3 to 4 times, and then non-administration period of at least 3 weeks is provided. This forms one course, and administration is repeated.

In the case of INGN-201, clinical tests are still proceeding and the optimal dose has not yet been established. However, no severe adverse effects have been observed up to 10¹¹ PFU (plaque forming units) in clinical tests performed in Japan and up to 3 x 10¹² VP (virus particles) in clinical tests performed in the United States. Intratumoral administration is preferable, but administration routes are not limited to this.

The antitumor effect of the pharmaceutical composition may be tested and examined as described below.

### (1) Examination of Combined Effect of Recombinant Virus and Substance Having Antitumor Effect

When the recombinant virus and the substance having an antitumor effect contained in the pharmaceutical composition are used in combination, antitumor effect is exerted against those tumors which are not affected by independent use of these substances. Therefore, by using the pharmaceutical composition, it becomes possible to treat a wider range of tumors.

Depending on the type of tumor, there are cases in which the antitumor effect of the pharmaceutical composition is not exerted when the substance having an antitumor effect is administered at a high concentration, though the antitumor effect of the pharmaceutical composition is exerted when the substance having an antitumor effect is administered at a low concentration. Since administration of substances having an antitumor effect often produces adverse effects, reduction of dose levels as much as possible is also desirable from the viewpoint of improving patients' QOL.

In order to analyze to what extent the pharmaceutical composition exerts an antitumor effect on cancer cells, XTT assay may be used, for example.

XTT (2,3-bis [2-Methoxy-4-nitro-5-sulfophenyl]- 2H-tetrazolium-5-carboxyanilide inner salt) assay is based on the measurement of the activity of viable cells by mitochondrial dehydrogenase in viable cells. It is a method suitable for monitoring cytotoxicity *in vitro*. Hereinbelow, XTT assay will be described specifically.

XTT solutions, without phenol red or dissolved in balanced salt solutions, are yellowish in color. When the XTT solution is contacted with viable cells, mitochondrial dehydrogenases of viable cells cleave the tetrazolium ring of XTT, yielding orange formazan crystals which are soluble in aqueous solutions. Actually, the bioreduction of XTT is inefficient. Therefore, an electron coupling agent such as phenazine methosulfate (PMS) is often added to the reaction solution. An increase or decrease in cell numbers results in a concomitant change in the amount of formazan formed. Therefore, it is possible to determine the degree of cytotoxicity caused by the substance of interest by measuring the resultant orange solution colorimetrically. In the analysis of the pharmaceutical composition, various cancer cells cultured *in vitro* are infected with the recombinant virus at appropriate concentrations (MOI: multiplicity of infection). The above cancer cells are cultured under appropriate conditions. Then, a substance having an antitumor effect is added to the culture broth of the virus-infected cells at various concentrations. The above-described XTT assay may be performed when cells have been cultured for an appropriate period of time after viral infection. Thus, antitumor effect can be analyzed.

### (2) Influence of Substance Having Antitumor Effect on Proliferation of Recombinant Virus

Even when the recombinant virus and the substance having an antitumor effect contained in the pharmaceutical composition are used in combination, the proliferation/replication of the recombinant virus is not affected at all by the presence of the substance having an antitumor effect. Therefore, the antitumor effect of the virus contained in the pharmaceutical composition exerted when used alone is not inhibited even when combined with another substance.

The influence of the substance having an antitumor effect combined in the pharmaceutical composition upon the proliferation of the recombinant virus in target cells may be measured, for example, as described below using quantitative real-time PCR. Briefly, the recombinant virus was grown in target cells for an appropriate period of time in the presence of the substance having an antitumor effect. Subsequently, virus-infected cells are harvested, followed by DNA extraction. The DNA is subjected to real-time PCR using primers targeting an appropriate gene carried by the virus. Thus, it is possible to quantitatively analyze the appropriate gene carried by the virus. At this time, if a gene encoding a fluorescent substance is integrated in the recombinant virus cells where viral proliferation occurred emit a specific fluorescence (e.g., green fluorescence when GFP is used) when exposed to excitation light. Therefore, viral proliferation within cells can be visualized. For example, when cells infected with virus are observed under fluorescence microscope, emission of GFP fluorescence in the cells can be observed. Further, by observing the emission of GFP fluorescence with the passage of time using a CCD camera, virus-infected cells can be observed with the passage of time. When such a recombinant virus is administered to the living body, it is also possible to label and detect cells *in vivo* in real time.

### (3) Influence of Substance Having Antitumor Effect and Recombinant Virus on the Cell Cycle of Tumor Cells

The antitumor effects produced by the substance having an antitumor effect and the recombinant virus used in combination in the pharmaceutical composition are independent from each other and do not affecting each other. The antitumor effect of the recombinant virus on tumor cells is produced by a mode of action completely different from a mode of action by which the substance having an antitumor effect produces its antitumor effect. Therefore, even in the presence of the recombinant virus, the substance having an antitumor effect does not lose its function of inhibiting the cell cycle of tumor cells and inducing apoptosis.

The influence of the substance having an antitumor effect and the recombinant virus used in combination in the pharmaceutical composition upon the cell cycle of target cells may be examined, for example, by analyzing cell cycle with a nuclear dye PI (propidium iodide) using flow cytometry.

Flow cytometry is a method of measuring individual sizes, DNA contents, etc. of cells in a cell population by feeding a cell suspension at a high speed and measuring fluorescence emitted by individual particles. Flow cytometry is capable of not only analyzing the relative sizes, shapes and difference in internal structures, but also performing cell identification and analyzing ratios of various cells constituting a cell group in a short time by determining fluorescence intensities and fluorescence types utilizing fluorescence labeling.

As a result of cell death, cell membranes become unable to retain their states and nuclei are readily stained with a nuclear dye such as PI mentioned above. Therefore, DNA content and cell cycle can be judged from the results of nuclear staining with PI. Here, it should be note that when ratios of S phase and G2/M phase in cell cycle are high in tumor cells, the malignancy is judged to be high. Therefore, when the results show that the ratio of G2/M phase is suppressed, it can be said that antitumor effect is produced.

### (4) In vivo Antitumor Effect at the Time of Combined Use of Substance Having Antitumor Effect and Recombinant Virus

When the substance having an antitumor effect and the recombinant virus which are components of the pharmaceutical composition are used in combination, an extremely high antitumor effect can be obtained compared to the administration of the recombinant virus alone or the administration of the substance having an antitumor effect alone.

Antitumor effect may be analyzed, for example, as described below by measuring tumor sizes.

Briefly, an appropriate tumor cell (e.g., H1299) is inoculated subcutaneously at the back of 5-week old nude mice. When the tumor has grown to 5-10 mm in size, an appropriate amount of Telomelysin is administered intratumorally and, after a while, an appropriate amount of docetaxel is administered intraperitoneally. At this time, PBS may be administered intratumorally and intraperitoneally as control. After an appropriate period of time from these administrations, tumor sizes are measured and analyzed as to whether a significant difference is observed compared to the control.

There may be cases where the substance having an antitumor effect has another effect of enhancing the infection efficiency of the recombinant virus. This effect can be confirmed, for example, by adding the substance having an antitumor effect to a culture broth of tumor cells, inoculating the recombinant virus into the culture broth, measureing to what extent the virus has infected the tumor cells, and comparing the measuring results with the state of infection of the virus inoculated into a culture broth of tumor cells not treated with the substance having an antitumor effect.

In order to examine to what extent the virus has infected tumor cells, a replication-incompetent, lacZ gene-expressing adenovirus prepared by integrating a lacZ gene in the replication-incompetent recombinant virus vector of the present invention may be used, for example. According to this method, when the replication-incompetent, lacZ gene-expressing adenovirus infects and proliferates in cells, lacZ gene is expressed and the cells develop a blue color. Thus, viral infection can be confirmed easily. Alternatively, a recombinant virus prepared by integrating a gene encoding a labeling protein (e.g., GFP) at an appropriate site of the genome of the recombinant virus may be used. With such a recombinant virus, it is possible to confirm viral proliferation by quantitatively determining GFP.

The combined effect of the substance having an antitumor effect and the recombinant virus may also be confirmed by tests using isobologram.

Isobologram is a system for analyzing the presence or absence of synergistic effect of two substances. Specifically, the presence or absence of synergistic effect of two drugs is determined by examining the progress of tumor cell proliferation based on MTT assay or thymidine intake. MTT assay is a sensitivity test for pharmaceutical compositions. Specifically, MTT assay is performed by culturing tumor cells mixed with various anticancer agents for an appropriate period of time, judging the activity of viable tumor cells at the end of the culture by judging the activity of mitochondrial succinic dehydrogenase (SD), and thereby judging the sensitivity to the anticancer agent. Briefly, tumor cells are reacted with a tetrazolium salt (MTT) which is a substrate for SD. Deposited formazan crystals are dissolved in DMSO, followed by measurement of absorbance of the purple color developed with a microplate reader (MTT judgment). By thus measuring the activity of viable cells, it has become possible to judge the effect of drugs by colorimetry. For example, the substance having an antitumor effect and the recombinant virus are administered to an appropriate tumor cell such as human lung cancer cell H1299. The cell is cultured and then subjected to MTT assay by the above described method. As a result, antitumor effect against H1299 can be measured.

### (5) Histological Changes in Tumor Tissue and Liver after Intratumoral Administration of the Pharmaceutical Composition of the Present Invention (HE Staining)

The pharmaceutical composition is an excellent pharmaceutical composition with high safety which would not affect the liver. When a pharmaceutical composition is administered to the body, it is degraded/detoxified (metabolized) in the liver, gastrointestinal mucosa, kidney, etc. At this time, the major metabolic organ is the liver. When the pharmaceutical composition is administered to the body, the composition exerts antitumor effect on target tumor tissues but does not affect the liver which is an important metabolic organ as described above. Therefore, the pharmaceutical composition can be said an excellent pharmaceutical composition with high safety.

Evaluation of the safety of pharmaceutical compositions may be performed by analyzing changes in tumor tissues and liver tissues after intratumoral administration of, for example, the pharmaceutical composition. Such changes may be confirmed by histological staining methods such as hematoxylin-eosin (HE) staining.

HE staining is a basic, histological staining method which utilizes that tissue samples are stained in different colors depending on electrical charges in tissues. For example, nuclei are stained in bluish purple, and other cytoplasms, fibers, erythrocytes, etc. are stained in various, light to dark red colors depending on their natures.

Specifically, tissue sections are prepared from tumor tissues to which the pharmaceutical composition has been administered; after deparaffinization, the tissue sections are treated with hematoxylin solution and then with eosin solution; thus, histological changes in tumor tissues can be confirmed. For example, when antitumor effect is observed in tumor cells, the tumor cells cause hyaline degeneration.

### 4. Inhibiting Tumor Cell Growth with the Pharmaceutical Composition

The above-described Telomelysin recombinant virus and the substance having an antitumor effect are used in combination to inhibit tumor cell growth.

For the inhibition of tumor cell growth, for example, a microtubule inhibitor may be administered after administration of Telomelysin. It is clear from experiments using cultured cells that a remarkable combined effect is achieved by this method. Alternatively, Telomelysin may be intratumorally administered and a microtubule inhibitor may be systemically administered at the same time. In animal experiments, an evident combined effect by the above-described simultaneous administration is recognized. When INGN-201 and Telomelysin are used in combination, sometimes it is effective to administer INGN-201 24 hours after the administration of Telomelysin, and sometimes simultaneous administration produces similar effect, depending on the type of tumor to be treated. While it is more effective to administer Telomelysin before INGN-201 in lung cancer, simultaneous administration produces similar effect in large bowel cancer. It should be noted here that the combined administration of INGN-201 and Telomelysin is not limited to these tumor cells.

Hereinbelow, the present invention will be described more specifically with reference to the following Examples. However, the present invention is not limited to these Examples.

### [EXAMPLE 1]

### <Preparation of Recombinant Adenovirus (Telomelysin) >

E1A gene fragment of 897 pb was amplified from RNA extracted from human embryonic kidney 293 cells by RT-PCR using the following specific primers (E1A-S: SEQ ID NO: 5 and E1A-AS: SEQ ID NO: 6) under the conditions described below.
E1A-S: 5'-ACA CCG GGA CTG AAA ATG AG-3' (SEQ ID NO: 5)
E1A-AS: 5'-CAC AGG TTT ACA CCT TAT GGC-3' (SEQ ID NO: 6)

Composition of the PCR solution:
   1 x PCR buffer
   0.2 mM each dNTP
   5 mM MgCl₂
   2.5 U AmpliTaq Gold
   0.2 µM each Primer
Reaction conditions:
   95°C, 10 min
   (95°C, 1 min; 56°C, 1 min; 72°C, 1.5 min) x 32 cycles 72°C, 7 min
   4°C, 5 min

In a similar manner, E1B gene fragment of 1822 bp was amplified from DNA extracted from 293 cells by DNA-PCR using the following primers (E1B-S: SEQ ID NO: 7 and E1B-AS: SEQ ID NO: 8). The composition of the PCR reaction solution and reaction conditions (cycles, temperatures) were the same as described for the amplification of E1A gene.
E1B-S: 5'-CTG ACC TCA TGG AGG CTT GG-3' (SEQ ID NO: 7)
E1B-AS: 5'-GCC CAC ACA TTT CAG TAC CTC-3' (SEQ ID NO: 8)

For each of the PCR products, TA Cloning (TA Cloning Kit Dual Promoter; Invitrogen) was performed to confirm sequences. Then, DNA fragments of 911 bp (E1A) and 1836 bp (E1B) were cut out with restriction enzyme EcoRI.

E1A and E1B were inserted into the MluI site and the SalI site, respectively, of pIRES vector (CLONTECH) in forward orientation (E1A-IRES-E1B).

A 455 bp hTERT promoter sequence which had been cut out with restriction enzymes MluI and BglII was inserted into the XhoI site located upstream of the E1A of E1A-IRES-E1B in forward orientation (phTERT-E1A-IRES-E1B).

The cytomegalovirus (CMV) promoter contained in pShuttle vector was removed by treatment with restriction enzymes MfeI and NheI. Then, a 3828 bp sequence cut out from phTERT-E1A-IRES-E1B using restriction enzymes NheI and NotI was inserted into that site (pSh-hAIB).

A 4381 bp sequence was cut out from pSh-hAIB using restriction enzymes I-CeuI and PI-SceI, and inserted into the Adeno-X Viral DNA of Adeno-X Expression System (CLONTECH) (AdenoX-hAIB). This AdenoX-hAIB was treated with restriction enzyme PacI for linearization and then transfected into 293 cells to thereby prepare an infectious recombinant adenovirus (Telomelysin). A schematic diagram of the replication cassette integrated in Telomelysin is shown in Fig. 1.

### [EXAMPLE 2]

### <Examination of Combined Effect of Telomelysin and Microtubule Inhibitor>

Telomelysin was infected to the following various cancer cells cultured *in vitro* at the concentrations of 0 (Mock), 0.1 or 1 MOI (multiplicity of infection). The cells used were as follows.
Human lung cancer cells (H1299, A549 and H226Br)
Human large bowel cancer cells (SW620 and DLD-1)
Human gastric cancer cell (MKN28)
Human esophagus cancer cells (TE8 and T.Tn)
Human liver cancer cell (HepG2)
Human prostate cancer cell (LNCaP)

Specifically, 1 x 10³ cells from each of the above cell strains were plated in a 96-well plate. After 24 hours, cells were counted and the virus was added to the culture broth at a concentration of 0.1 MOI or 1 MOI.

On the next day, docetaxel was added to the virus-infected cell culture broth at various concentrations (control, 0.1 nM, 1.0 nM, 10.0 nM and 100.0 nM).

On day 5 of infection, XTT assay was performed using a kit (Roche Diagnostics). Briefly, the culture broth was removed from the plate. A reaction solution containing XTT reagent was prepared and added to the plate. Cells were cultured for about 4 hours. Then, absorbance was measured with a microtiter plate (ELISA) reader, and the number of viable cells was calculated to thereby confirm antitumor effect.

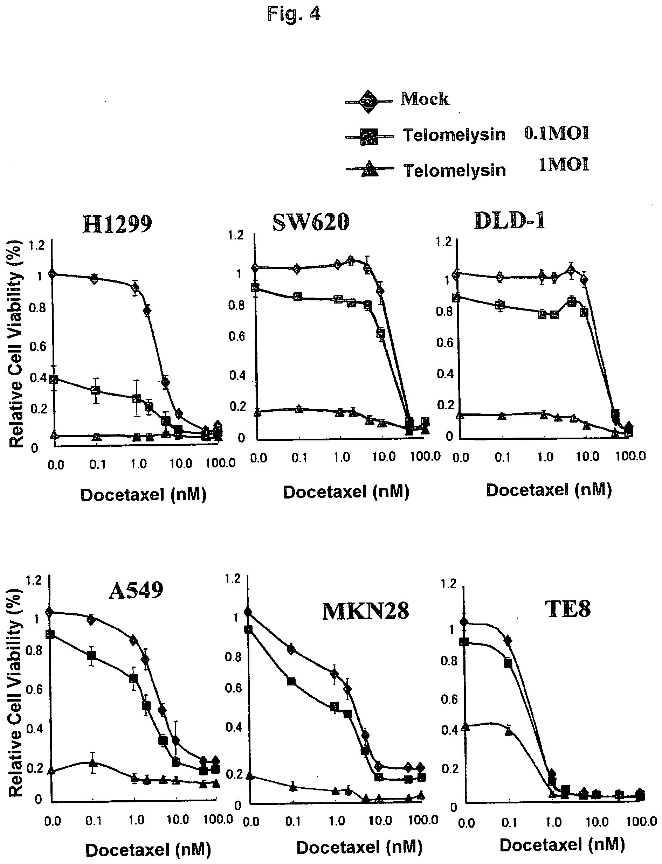

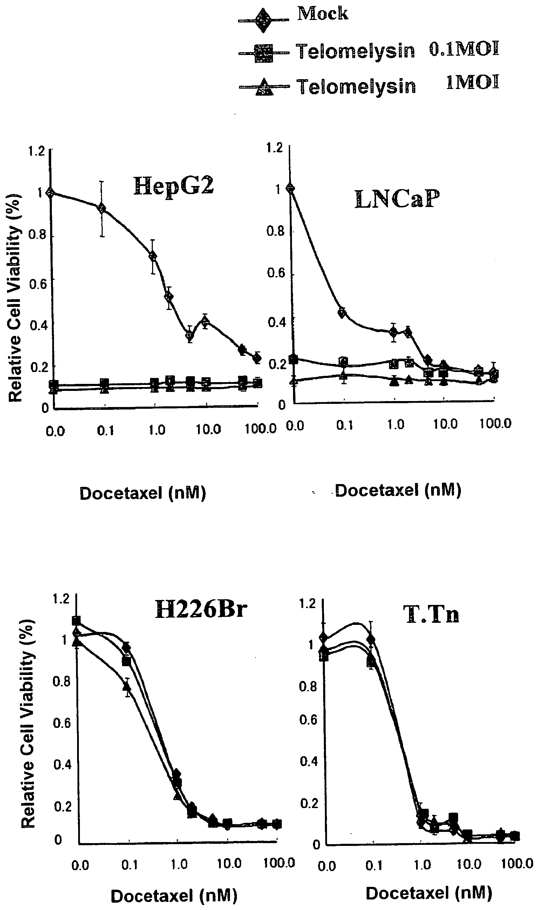

The results are shown in Fig. 4. Combined effect of Telomelysin 0.1 MOI and low concentrations of docetaxel was recognized in the cancer cell strains indicated in the upper row in Fig. 4. Among all, combined effect was especially remarkable in H1299 cell. On the other hand, evident combined effect was not observed in cell strains such as HepG2 and LNCaP with high sensitivity to Telomelysin and cell strains such as H226Br and T.Tn with sensitivity to docetaxel itself.

### <Examination of Combined Effect of Telomelysin and Other Anticancer Agents>

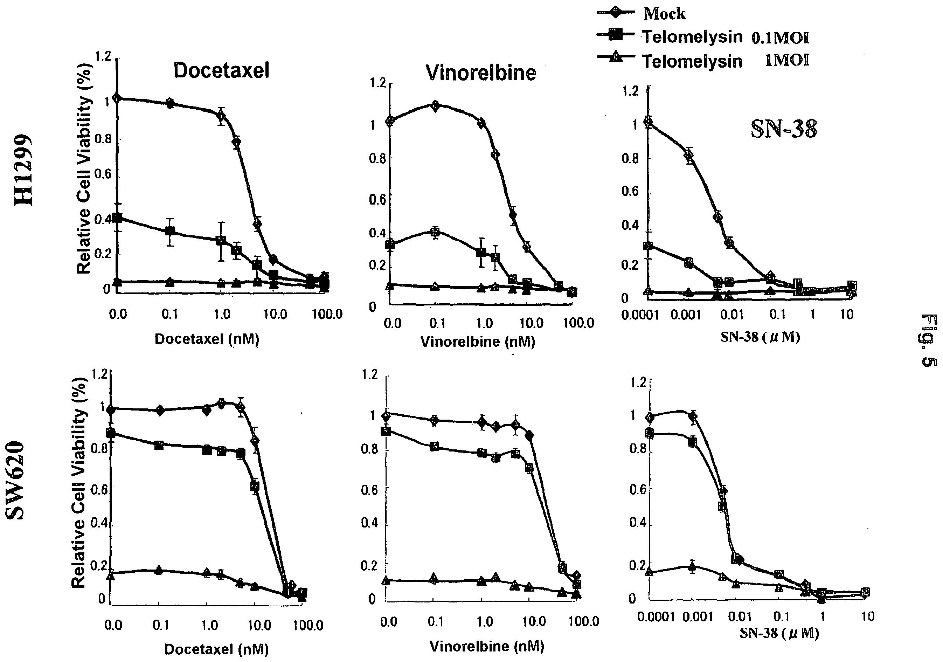

XTT assays were performed in the same manner as described above using microtubule inhibitor vinorelbine and topoisomerase I inhibitor SN-38 (CPT-11 metabolite) as anticancer agents combined with Telomelysin. As a result, vinorelbine showed combined effect almost equal to that shown by docetaxel. Any of the cell strains tested has sensitivity to SN-38, and combined effect was observed when the concentration of SN-38 was low (Fig. 5).

### <Influence of Anticancer Agent upon the Proliferation of Recombinant Virus>

How the proliferation of Telomelysin would be affected when an anticancer agent acting on microtubule is combined with Telomelysin was analyzed by quantitative real-time PCR.

Human lung cancer cell H1299 and human large bowel cancer cell SW620 were infected with Telomelysin at 0.1 MOI for 2 hours. Two hours thereafter, docetaxel was administered. At 12, 24, 36, 48 and 60 hours after the infection, cells were harvested. DNA contained in the cells and in the supernatant was extracted separately. Real-time PCR was performed using primers targeting the E1A gene carried by Telomelysin, to thereby analyze viral proliferation/replication quantitatively. Specifically, 2 µl of DNA extract was added to 18 µl of PCR solution. PCR was performed using E1A primers under the following conditions.
E1A primer sequences:
   (Forward) 5'-CCTGTGTCTAGAGAATGCAA -3' (SEQ ID NO: 9)
   (Reverse) 5'-ACAGCTCAAGTCCAAAGGTT -3' (SEQ ID NO: 10)
Composition of the PCR solution: 1×LC FastStart DNA Master SYBR Green I
   3mM MgCl₂
   0.5 µM each primer
Reaction conditions: 95°C, 10 min
   (95°C, 10 sec; 60°C, 15 sec; 72°C, 8 sec) x 40 cycles
   70°C, 15 sec
   40°C, 30 sec

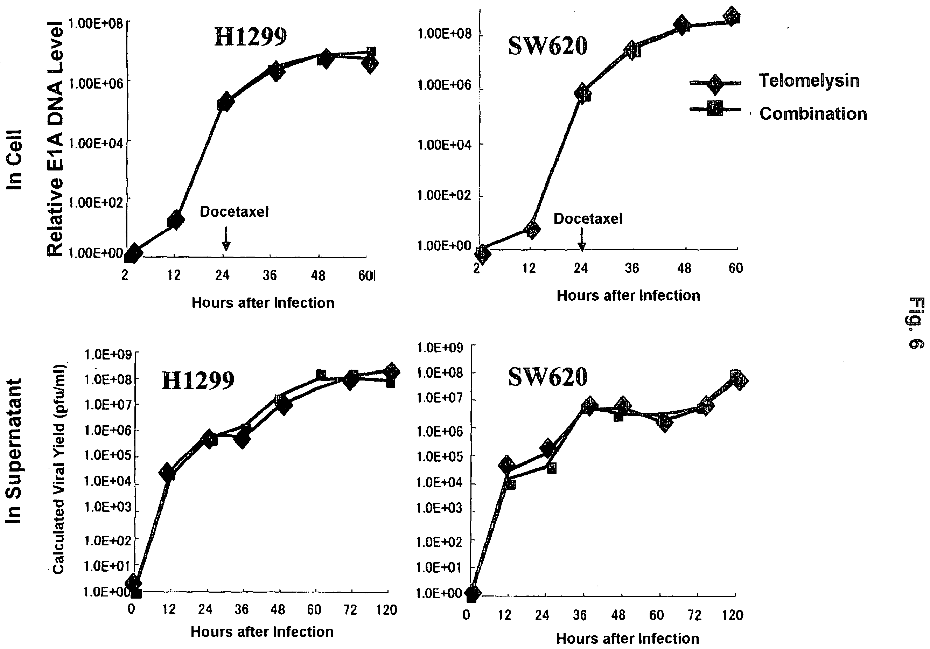

The results are as described below (Fig. 6).
a) Telomelysin proliferated in cells rapidly over 12-24 hours. Similar proliferation efficiency was observed when docetaxel treatment was performed.
b) Telomelysin diffused in supernatant exhibited equal proliferation efficiency when used alone and when used in combination. Since all the supernatants were collected and subjected to DNA extraction, it is believed that these results reflect the total amount of virus released into the supernatant as a result of cell death.

From these results, it is believed that combined use of an anticancer agent does not affect the proliferation/replication of Telomelysin and does not inhibit the antitumor effect of the virus (Fig. 6).

### <Influence of Telomelysin and Microtubule Inhibitor upon the Cell Cycle of Tumor Cells>

Cell cycle was analyzed with PI (propidium iodide) using flow cytometry.

As a result, no change was observed in cell cycle 12 and 48 hours after treatment with Telomelysin alone at 0.1 MOI.

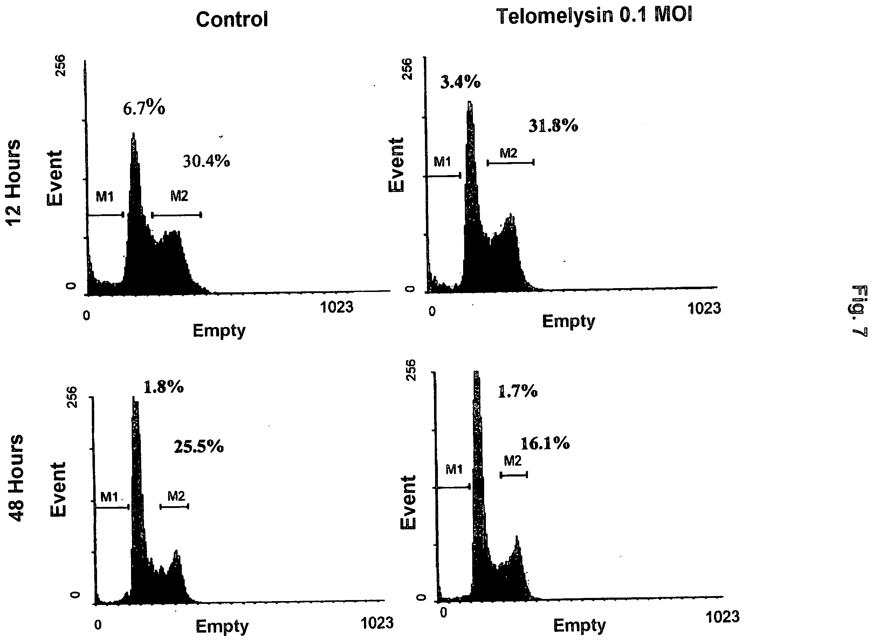

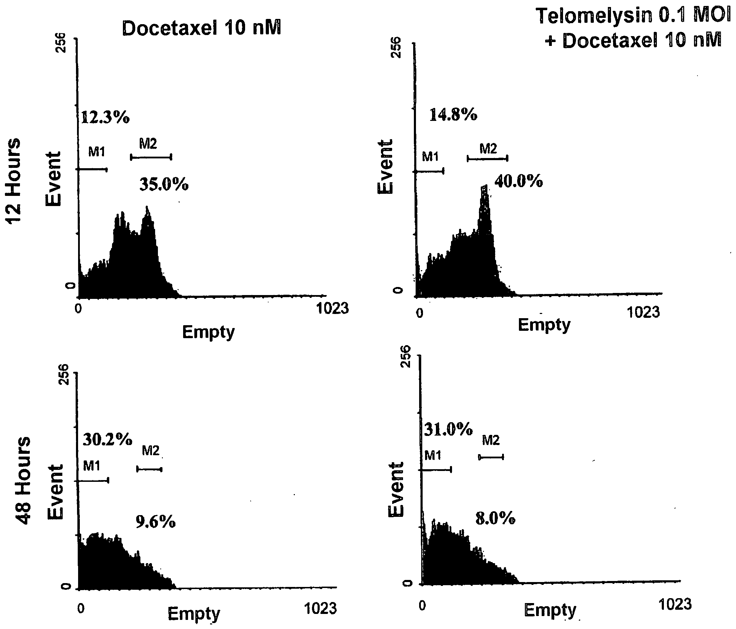

When docetaxel was administered alone or Telomelysin and docetaxel were administered in combination, cells began to exhibit docetaxel-dependent G2/M arrest 12 hours after administration; then, sub G0/G1 increased gradually; and apoptosis (DNA fragmentation) which seemed to be docetaxel-dependent was observed (Fig. 7).

From these results, it is believed that the antitumor effect caused by Telomelysin is different from apoptosis caused by docetaxel in molecular mechanism.

### <In vivo Antitumor Effect of Telomelysin and Microtubule Inhibitor in H1299 Lung Cancer Cell>

H1299 was inoculated subcutaneously at the back of 5-week old nude mice. One, three and five days after the tumor reached 5-10 mm in size, Telomelysin was administered intratumorally at a dose of 1 x 10⁷ pfu/50 µl/mouse and docetaxel was administered at a dose of 12.5 mg/kg intraperitoneally both three times in the total. As a control, PBS was administered intratumorally and intraperitoneally. Tumor size was measured in every three days.

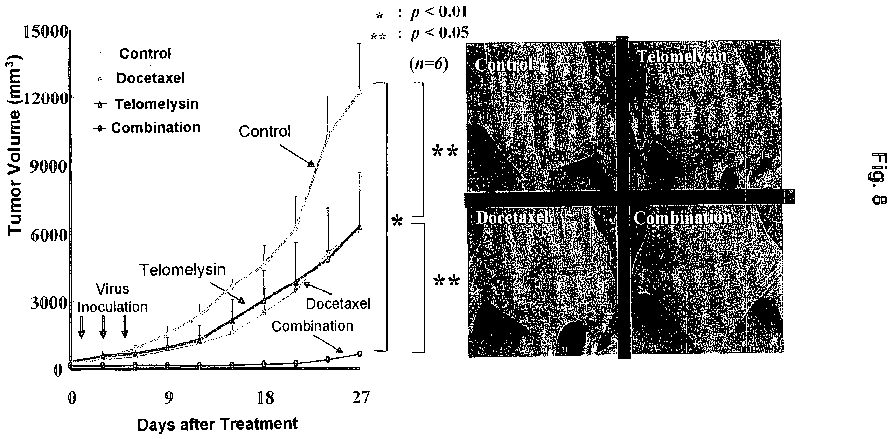

As a result, although a significant growth inhibition was observed in the Telomelysin alone group compared to the control group, a still more significant antitumor effect was observed in the combination group compared to the control group and single use groups (Fig. 8).

### <Histological Changes in Tumor Tissue and Liver after Intratumoral Administration of Telomelysin (HE Staining)>

H1299 was inoculated subcutaneously at the back of 5-week old nude mice. When the tumor reached 5-10 mm in size, Telomelysin was administered intratumorally. Six days after the final administration, tumor tissue and liver were stained with HE as described below. Briefly, tissue sections were prepared, deparaffinized and washed with water. Then, distilled water was fed to the tissue section. Hematoxylin solution was added thereto and left stationary for 20 minutes. Then, the section was rinsed with water lightly. The resultant section was fractionated with 1% hydrochloric acid/70% alcohol, followed by color development. Then, the tissue section was washed with water for 10 minutes, passed through 80% ethanol, and dipped in eosin solution for 10 minutes. Subsequently, the tissue section was washed with water lightly, dehydrated, cleared and mounted to thereby perform HE staining.

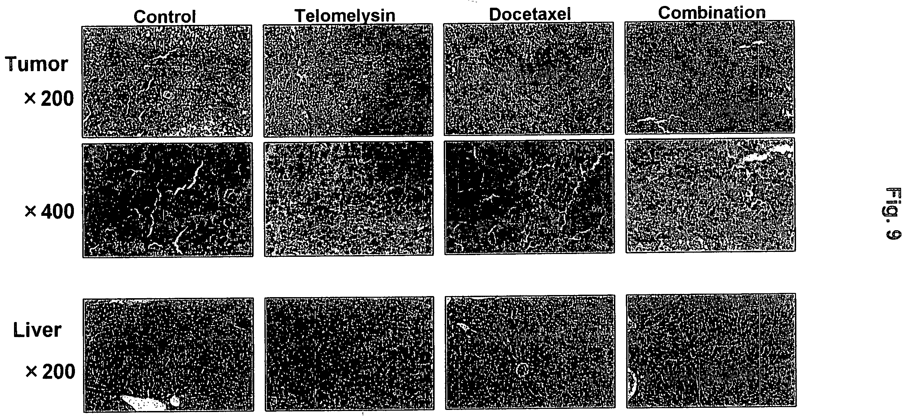

As a result, hyaline degeneration of tumor cells was observed in a wide range when Telomelysin was administered alone or in combination (see Fig. 9, panels titled "Telomelysin" and "Combination"). Toxicity was not observed in any of the liver tissues examined (Fig. 9).

### [EXAMPLE 3]

### <Enhancement of the Expression of Adenovirus Receptor CAR by HDAC Inhibitor FR901228>

CAR expression was analyzed by adding FR901228 (Fujisawa Pharmaceutical Co., Ltd.) to culture broths of various human lung cancer cells (A549, H358, H460 and H1299) at 1 ng/ml, treating the cells for 48 hours, and subjecting the cells to flow cytometry.

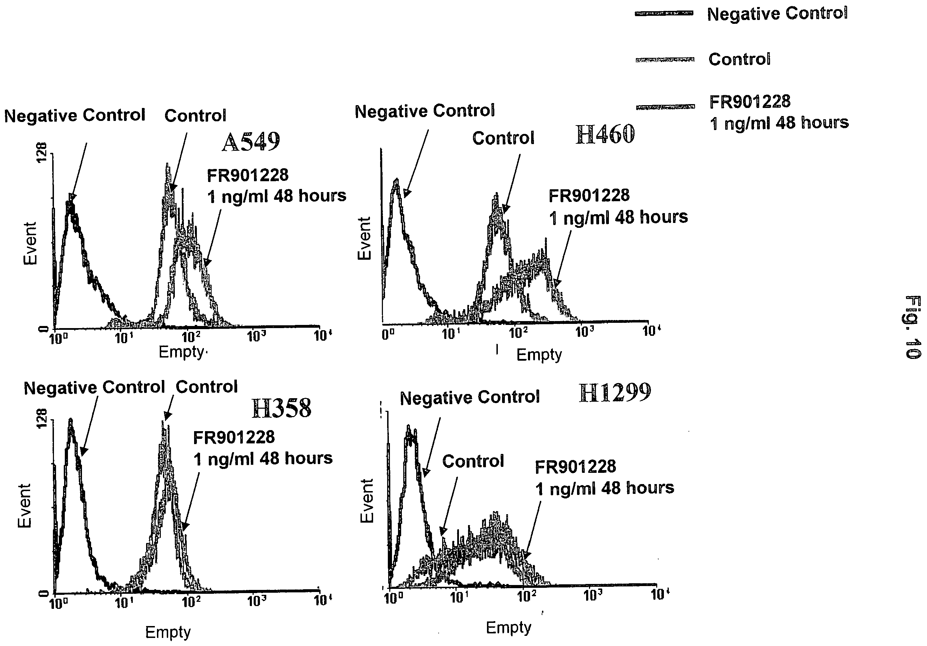

The results revealed that CAR expression was enhanced in A549 and H460 cells, but no change was observed in H358 and H1299 cells (Fig. 10).

### <Enhancement of Adenovirus Infection Efficiency by HDAC Inhibitor FR901228>

Subsequently, whether HDAC inhibitor FR901228 enhances adenovirus infection efficiency or not was examined using A549 lung cancer cell and a replication-incompetent, lacZ gene-expressing adenovirus. FR901228 was added to culture broth of A549 lung cancer cell at concentrations of 0.1 ng/ml and 1 ng/ml. After 48-hour treatment, cells were infected with the replication-incompetent, lacZ gene-expressing adenovirus at 1 MOI and 10 MOI, and the states of cells were observed.

When the adenovirus infected cells and proliferated therein, lacZ gene is expressed and the cells develop a blue color. By utilizing this phenomenon, enhancement of adenovirus infection efficiency by FR901228 was confirmed.

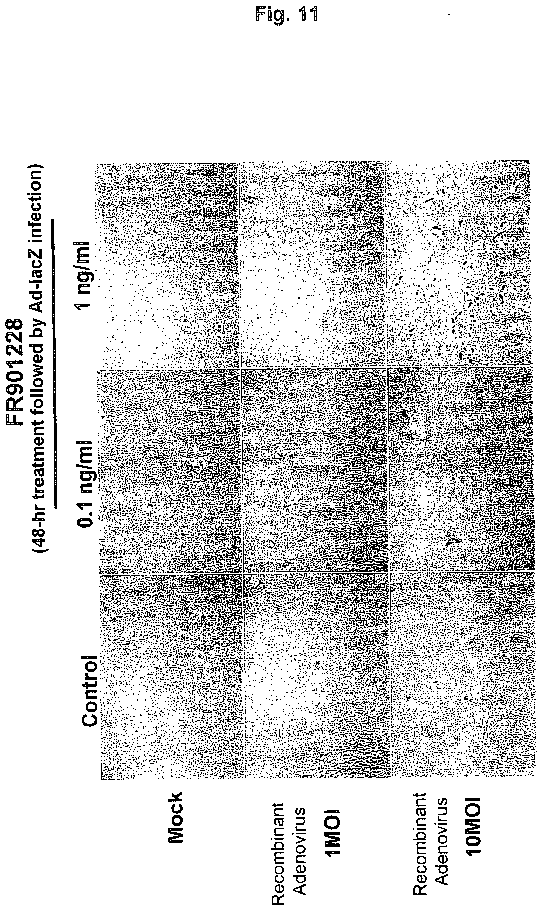

Since blue zones were observed dose-dependently when FR901228 treatment was performed, enhancement of lacZ expression was demonstrated. Therefore, it was confirmed that adenovirus infection efficiency is enhanced in a dose-dependent manner (Fig. 11).

### <Enhancement of Telomelysin Infection Efficiency by HDAC Inhibitor FR901228>

Whether HDAC inhibitor FR901228 enhances the infection efficiency of Telomelysin of the present invention or not was examined using A549 lung cancer cell and a replication-incompetent, lacZ gene-expressing adenovirus. The Telomelysin used in this experiment was a recombinant virus OBP-401 which was prepared by integrating a gene encoding a labeling protein GFP and a promoter regulating the expression of this gene in E3 region of the genome of the recombinant adenovirus of the present invention. With this virus OBP-401, enhancement of viral proliferation by FR901228 was confirmed using GFP expression as an indicator.

FR901228 was added to culture broth of A549 lung cancer cell at concentrations of 0.5 ng/ml and 1 ng/ml. After 48-hour treatment, cells were infected OBP-401 at 0.1 MOI.

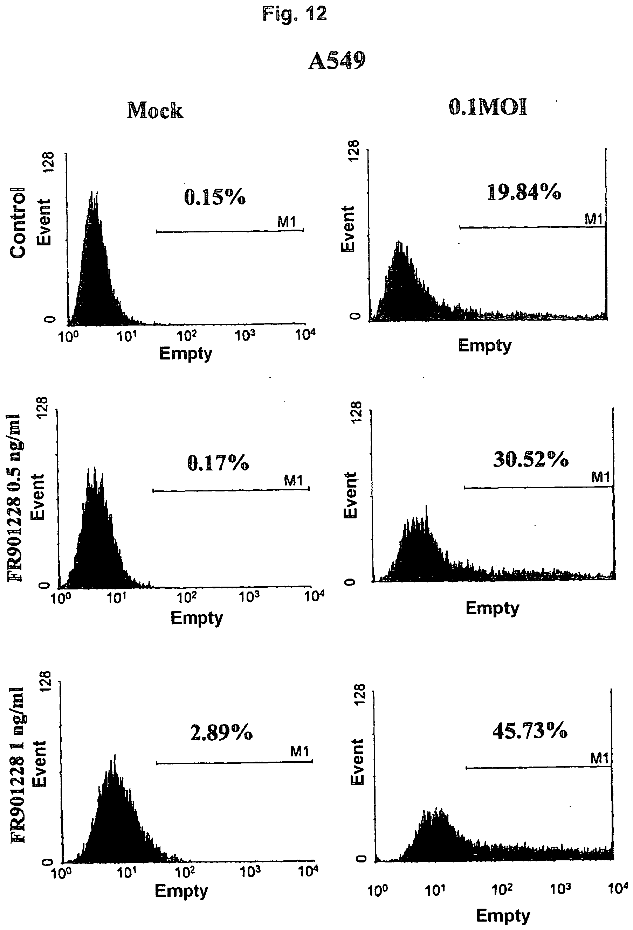

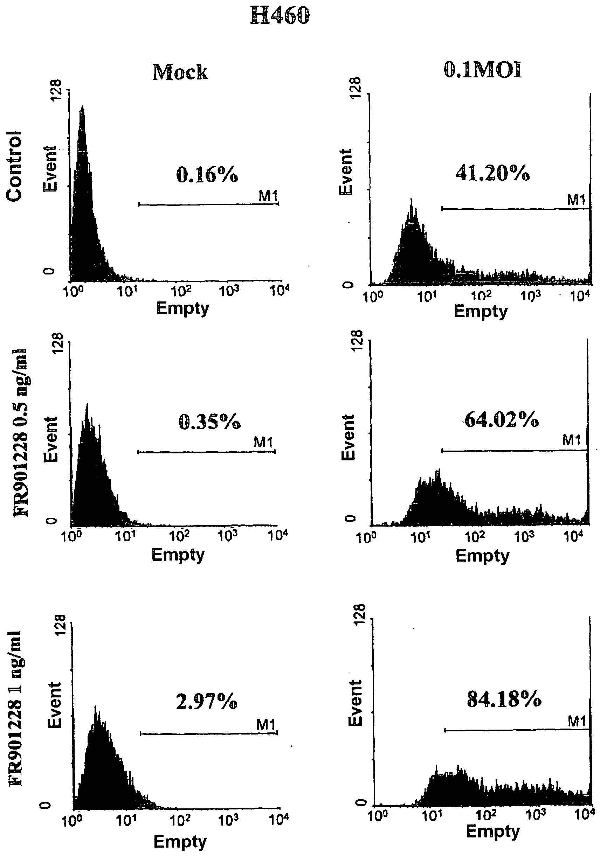

At 72 hours from the infection with OBP-401, GFP expression was quantitatively determined by flow cytometry. The results revealed that enhancement of GFP expression by FR901228 treatment was observed in a dose-dependent manner. Thus, it was confirmed that FR901228 enhances the infection and replication of OBP-201 (Fig. 12).

### <Enhancement of the Antitumor Effect of Telomelysin by HDAC Inhibitor FR901228>

In order to confirm the combined effect of FR901228 and Telomelysin, the synergistic effect of FR901228 and Telomelysin was examined with using isobologram (a system for analyzing the presence or absence of synergistic effect of two substances).

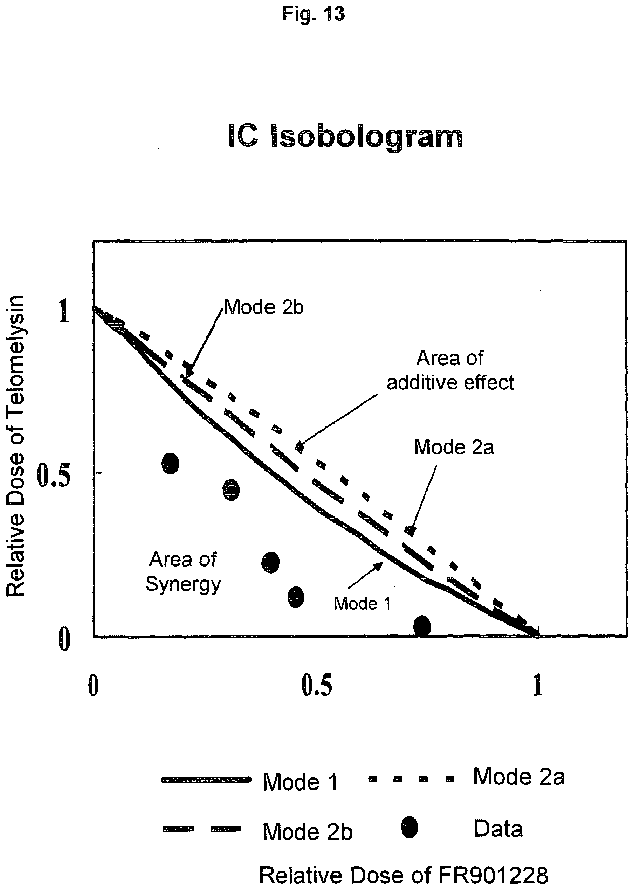

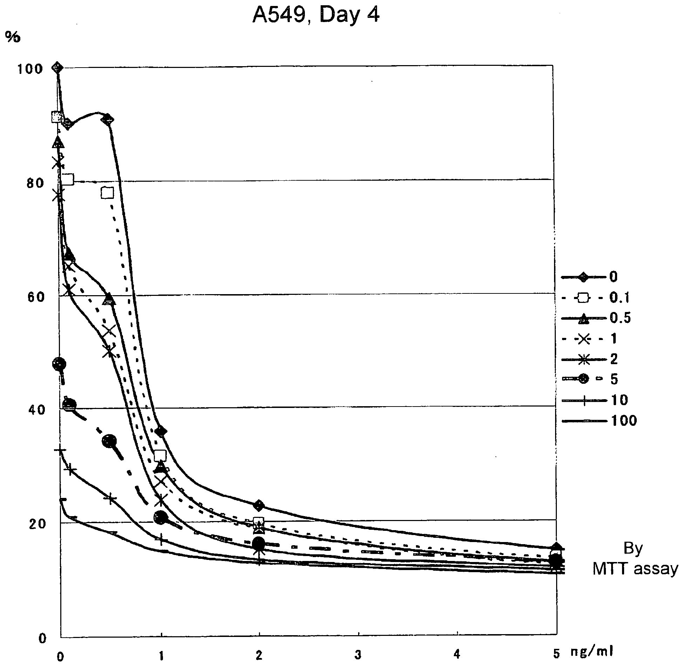

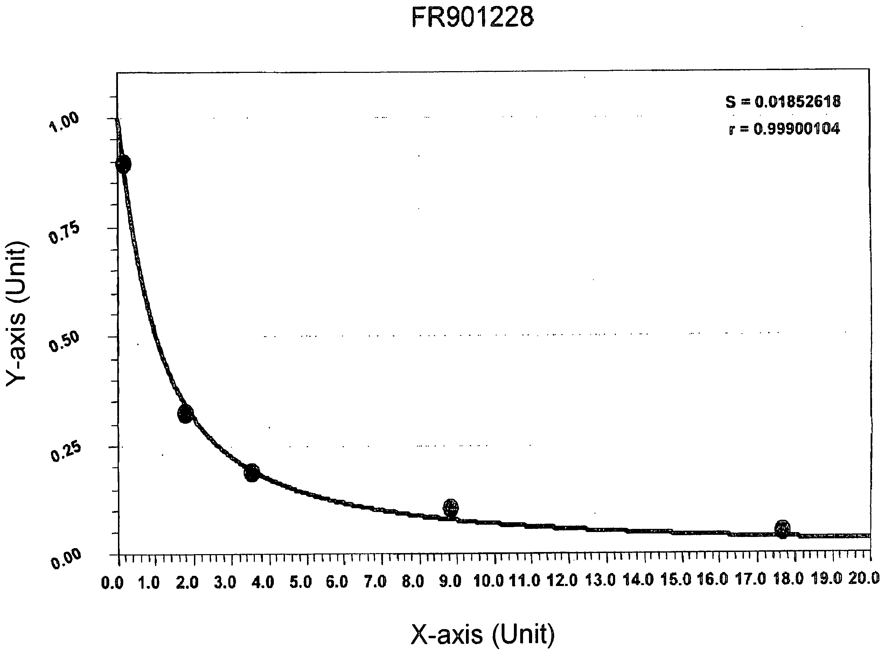

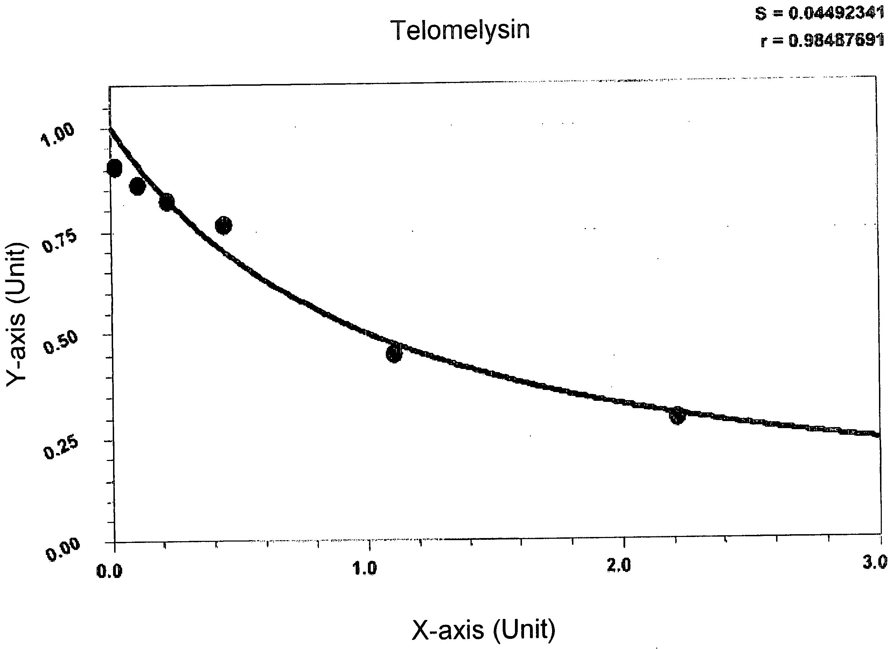

In this experiment, recombinant virus OBP-401 in which a gene encoding a labeling protein GFP and a promoter regulating the expression of this gene are integrated was used as Telomelysin, together with FR901228. Human lung cancer cell A549 cultured in 96-well plates was infected with Telomelysin at 0.1, 0.5, 1, 2, 5, 10 and 100 MOI. Simultaneously, FR901228 was added to cells at 0.01, 0.5, 1, 2 and 5 ng/ml. On day 4 of infection, viable cell count was determined by MTT assay. For MTT assay, culture broth was removed from each well. After washing with PBS, culture broth containing 0.5 mg/ml of MTT (Sigma) was added to each well. Then, cells were cultured for 4 hours. After addition of 0.04 N hydrochloric acid-containing isopropyl alcohol, absorbance was determined with a microplate reader. The upper right panel of Fig. 13 is a diagram in which all of the measured values are plotted. In the right lower panel where cell viability is plotted in the vertical axis and individual concentrations are plotted in the horizontal axis, dose-dependent antitumor effects of Telomelysin and FR901228 can be confirmed. The measured values were analyzed by the isobologram method of Steel and Peckham (Steel G. G. and Peckham M. J., Exploitable mechanisms in combined radiotherapy-chemotherapy: the concept of additivity. Int. J. Radiat. Oncol. Biol. Physiol., 5: 85-91, 1979) to thereby prepare the left panel of Fig. 13. The area below the blue line in mode 1 represents an area of synergy; all of the measured values are plotted within this area. Thus, it has become clear that FR901228 and Telomelysin act synergistically in human lung cancer cell H1299 (Fig. 13).

### [EXAMPLE 4]

### <Measurement of the Antitumor Effect of Gene Therapeutic Agent INGN-201 (Advexin)>

INGN-201 (hereinafter and in drawings, sometimes referred to as "Advexin") was infected to H1299, SW620 and TE8 cells cultured *in vitro* at a concentration of 0 (Mock), 10, 50, 100, 500 or 1000 MOI. Specifically, 1 x 10³ cells from each of the above cell strains were plated in a 96-well plate. After 24 hours, cells were counted, and Advexin was added to the culture broth at the concentrations indicated above. On day 5 of infection, XTT assay was performed using a kit (Roche Diagnostics). Briefly, the culture broth was removed from the plate. A reaction solution containing XTT reagent was prepared and added to the plate. Cells were cultured for about 4 hours. Then, absorbance was measured with a microtiter plate (ELISA) reader, and the number of viable cells was calculated to thereby confirm antitumor effect. The ratios of viable cells on day 5 were calculated, taking that ratio in non-treatment group as 1.0.

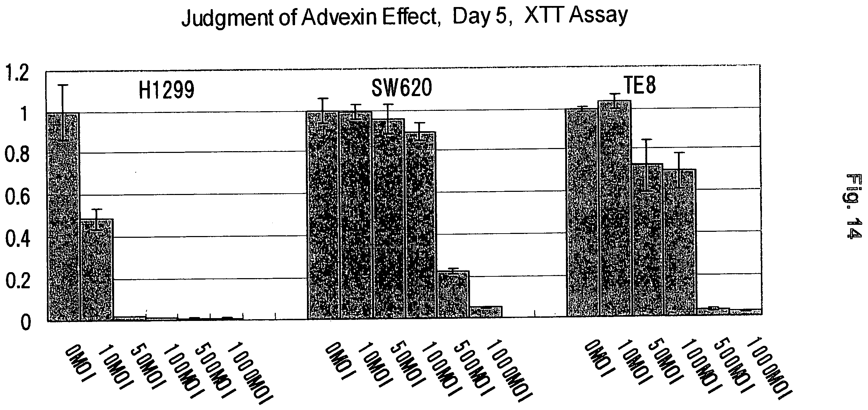

The results are shown in Fig. 14. Although remarkable antitumor effect was observed at 50 MOI in H1299 cell, antitumor effect was only recognized at 500 MOI or more in SW620 cell. In TE8 cell, although a slight inhibition of growth was recognized at 50 MOI, clear antitumor effect was only recognized at 500 MOI or more. Therefore, it is believed that H1299 is a high sensitivity strain; SW620 is a low sensitivity strain; and TE8 has a medium sensitivity.

### <Measurement of the Antitumor Effect by Simultaneous Administration of Telomelysin-Advexin>

Telomelysin at a concentration of 0, 1, 5 or 10 MOI and Advexin at a concentration of 0, 10, 50 or 100 MOI were administered in combination to H1299 and SW620 cells cultured *in vitro*. Specifically, 1 x 10³ cells from each of the above cell strains were plated in a 96-well plate. After 24 hours, cells were counted, and Telomelysin and Advexin were added to the culture broth at the concentrations indicated above. On day 5 of infection, XTT assay was performed using a kit (Roche Diagnostics). Briefly, the culture broth was removed from the plate. A reaction solution containing XTT reagent was prepared and added to the plate. Cells were cultured for about 4 hours. Then, absorbance was measured with a microtiter plate (ELISA) reader, and the number of viable cells was calculated to thereby confirm antitumor effect.

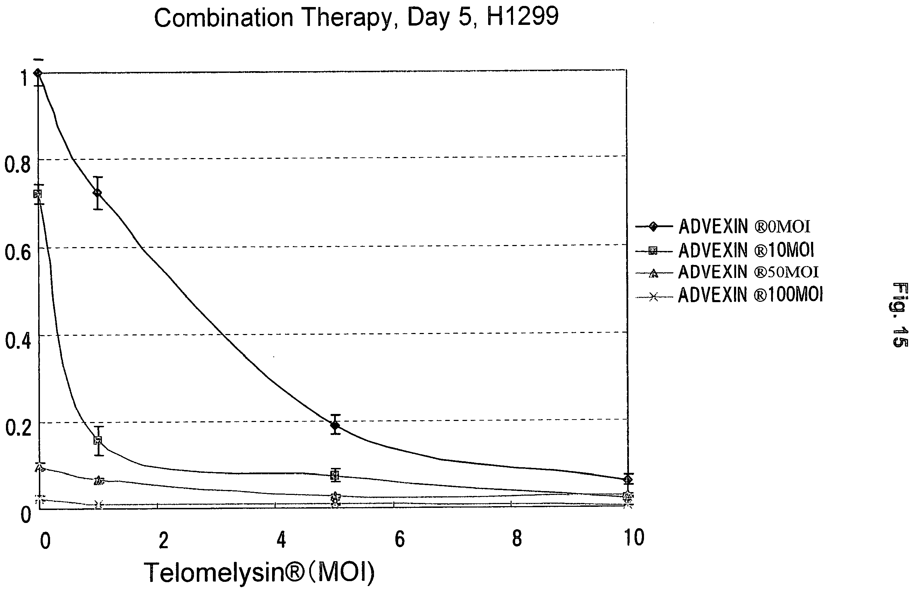

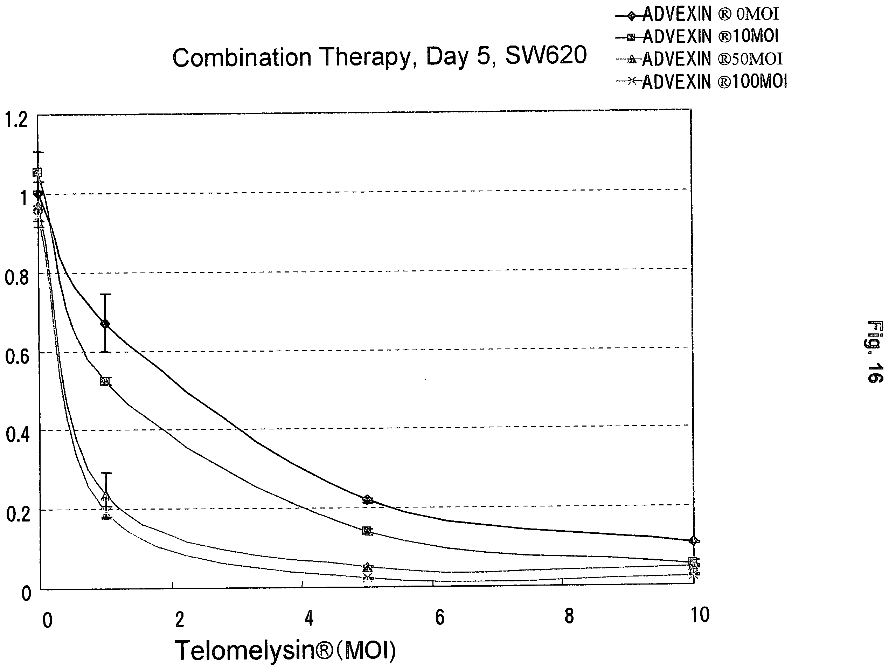

The results are shown in Figs. 15 and 16. In H1299 cell, combined use of 1 MOI Telomelysin and 10 MOI Advexin caused remarkable decrease in viable cell count and thus showed a remarkable antitumor effect (Fig. 15). Considering that antitumor effect began to appear at about 50 MOI when Advexin was used alone in H1299 cell (Fig. 14), these results show that a synergistic antitumor effect is obtained from combined use of Telomelysin and Advexin in H1299 cell. In SW620 cell, combined use of 1 MOI Telomelysin and 50 MOI Advexin caused remarkable decrease in viable cell count and thus showed a remarkable antitumor effect (Fig. 16). Considering that antitumor effect began to appear at about 500 MOI when Advexin was used alone in SW620 cell (Fig. 14), these results show that a synergistic antitumor effect is obtained from combined use of Telomelysin and Advexin in SW620 cell.

### <Measurement of the Antitumor Effect by Non-Simultaneous Administration of Telomelysin-Advexin>

H1299 and SW620 cells (1 x 10³ cells for each) were plated in 96-well plates, respectively, and cultured *in vitro*. After 24 hours, cells were counted. Then, (i) Telomelysin and Advexin were administered simultaneously to the culture broth; (ii) Advexin was administered 24 hours after the administration of Telomelysin (Telomelysin preceding); or (iii) Telomelysin was administered 24 hours after the administration of Advexin (Advexin preceding), to thereby infect cells. For comparison, Mock group was provided.

Specific concentrations of Telomelysin and Advexin used in combinations were as described below.
H1299 cell: 1 MOI Telomelysin and 5 MOI or 10 MOI Advexin
SW620 cell: 1 MOI Telomelysin and 10 MOI, 50 MOI or 100 MOI Advexin

Five days after the first infection, XTT assay was performed using a kit (Roche Diagnostics). Briefly, the culture broth was removed from the plate. A reaction solution containing XTT reagent was prepared and added to the plate. Cells were cultured for about 4 hours. Then, absorbance was measured with a microtiter plate (ELISA) reader, and the number of viable cells was calculated to thereby confirm antitumor effect.

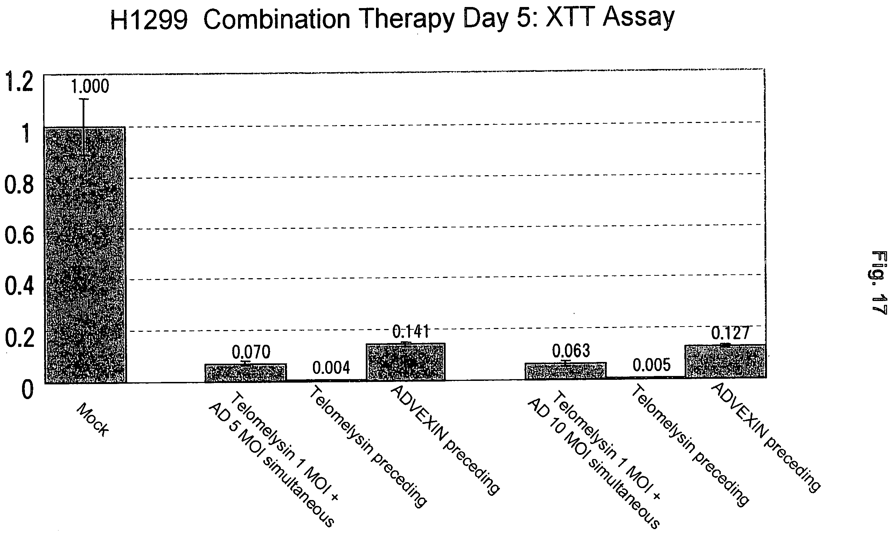

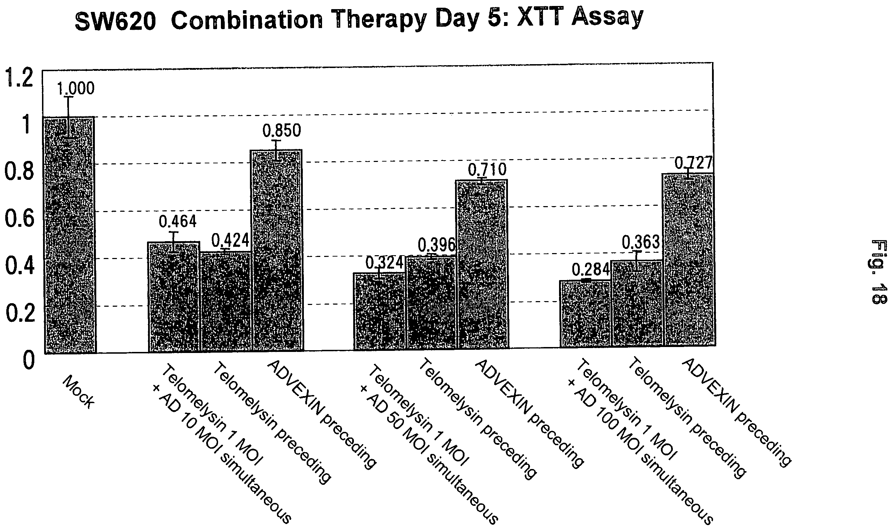

The results are shown in Figs. 17 and 18. In H1299 cell, a remarkable antitumor effect was obtained as compared to Mock, when any of the above-described methods (i) to (iii) was used (Fig. 17). Among all, the highest antitumor effect was obtained when administration of Telomelysin preceded. In SW620 cell, although an antitumor effect was observed in any of the above-described methods (i) to (iii), the antitumor effects in (i) and (ii) above were especially remarkable.

### INDUSTRIAL APPLICABILITY

According to the present invention, a pharmaceutical composition for use in combination therapy for tumors, comprising a recombinant adenovirus comprising a polynucleotide containing a promoter for human telomerase, an E1A gene, an IRES sequence and an E1B gene in this order, and a substance having an antitumor effect is provided. According to the pharmaceutical composition, an antitumor effect superior to the effect obtained by administration of the recombinant virus alone or the substance having an antitumor effect alone can be obtained, and thus it is possible to reduce the dose levels of the drugs used. Consequently, adverse effects caused by administration of anticancer agents can be inhibited.

Further, the pharmaceutical composition produces an antitumor effect even in those tumor cells in which independent use of the recombinant virus or the substance having an antitumor effect does not produce much effect.

Thus, anticancer treatment for more diversified tumors becomes possible.

### SEQUENCE LISTING FREE TEXT

SEQ ID NO: 5: Primer
SEQ ID NO: 6: Primer
SEQ ID NO: 7: Primer
SEQ ID NO: 8: Primer
SEQ ID NO: 9: Primer
SEQ ID NO: 10: Primer

## Claims

1. A pharmaceutical composition for use in a method of treatment of tumors by combination therapy, comprising a recombinant adenovirus comprising a polynucleotide containing a promoter for human telomerase, an E1A gene, an IRES sequence and an E1B gene in this order, and a substance having an anti-tumor effect, wherein the substance having an anti-tumour effect is vinorelbine, a topoisomerase I inhibitor, INGN-201 or a substance having antimetabolic activity.

2. The pharmaceutical composition for use according to claim 1, wherein the adenovirus comprises a said polynucleotide containing a hTERT promoter as the said promoter.

3. The pharmaceutical composition for use according to claim 1 or 2, wherein the substance having an antimetabolic activity is a folate, a purine, a pyrimidine, fluorouracil, tegafur or carmofur.

4. The pharmaceutical composition for use according to any one of the preceding claims, wherein the tumor is at least one selected from lung cancer, colon cancer, gastric cancer, breast cancer, esophageal cancer, head and neck cancer, liver cancer, pancreatic cancer, gallbladder/bile duct cancer, prostate cancer, bladder cancer, cervical cancer, thyroid cancer, ovarian cancer, leukemia, lymphoma, sarcoma and mesenchymal tumor.

5. A recombinant adenovirus comprising a polynucleotide containing a promoter for human telomerase, an E1A gene, an IRES sequence and an E1B gene in this order for use in a method of treating tumors in combination with a substance having an anti-tumour effect selected from vinorelbine, a topoisomerase I inhibitor, INGN-201 or a substance having antimetabolic activity.

6. A substance having an anti-tumour effect selected from vinorelbine, a topoisomerase I inhibitor, INGN-201 or a substance having antimetabolic activity for use in a method of treating tumors in combination with a recombinant adenovirus comprising a polynucleotide containing a promoter for human telomerase, an E1A gene, an IRES sequence and an E1B gene in this order.

7. The adenovirus for use according to claim 5, or the substance for use according to claim 6, wherein the adenovirus comprises a said polynucleotide containing a hTERT promoter as the said promoter.

8. The adenovirus for use according to claim 5 or 7, or the substance for use according to claim 6 or 7, wherein the substance having an antimetabolic activity is a folate, a purine, a pyrimidine, fluorouracil, tegafur or carmofur.

9. An adenovirus for use according to claim 5, 7 or 8, or a substance for use according to claim 6, 7 or 8, wherein the tumor is at least one selected from lung cancer, colon cancer, gastric cancer, breast cancer, esophageal cancer, head and neck cancer, liver cancer, pancreatic cancer, gallbladder/bile duct cancer prostate cancer, bladder cancer, cervical cancer, thyroid cancer, ovarian cancer, leukaemia, lymphoma, sarcoma and mesenchymal tumor.

10. Use of a recombinant adenovirus comprising a polynucleotide containing a promoter for human telomerase, an E1A gene, an IRES sequence and an E1B gene in this order for the manufacture of a medicament for use in a method of treating tumors in combination with a substance having an anti-tumour effect selected from vinorelbine, a topoisomerase I inhibitor, INGN-201 or a substance having antimetabolic activity.

11. Use of a substance having an anti-tumour effect selected from vinorelbine, a topoisomerase I inhibitor, INGN-201 or a substance having antimetabolic activity for the manufacture of a medicament for use in a method of treating tumors in combination with a recombinant adenovirus comprising a polynucleotide containing a promoter for human telomerase, an E1A gene, an IRES sequence and an E1B gene in this order.

12. Use of an adenovirus according to claim 10, or use of a substance according to claim 11, wherein the adenovirus comprises a said polynucleotide containing a hTERT promoter as the said promoter.

13. Use of an adenovirus according to claim 10 or 12, or use of a substance according to claim 11 or 12, wherein the substance having an antimetabolic activity is a folate, a purine, a pyrimidine, fluorouracil, tegafur or carmofur.

## Patentansprüche

1. Pharmazeutische Zusammensetzung zur Verwendung in einem Verfahren zur Behandlung von Tumoren durch Kombinationstherapie, umfassend ein rekombinantes Adenovirus, das ein Polynucleotid umfasst, welches einen Promotor für humane Telomerase, ein E1A-Gen, eine IRES-Sequenz und ein E1B-Gen in dieser Reihenfolge enthält, und eine Substanz, die Antitumorwirkung hat, wobei die Substanz, die Antitumorwirkung hat, Vinorelbin, ein Topoisomerase-I-Inhibitor, INGN-201 oder eine Substanz mit antimetabolischer Aktivität ist.

2. Pharmazeutische Zusammensetzung zur Verwendung gemäß Anspruch 1, wobei das Adenovirus ein Polynucleotid, das einen hTERT-Promotor als den Promotor enthält, umfasst.

3. Pharmazeutische Zusammensetzung zur Verwendung gemäß Anspruch 1 oder 2, wobei die Substanz mit antimetabolischer Aktivität ein Folat, ein Purin, ein Pyrimidin, Fluoruracil, Tegafur oder Carmofur ist.

4. Pharmazeutische Zusammensetzung zur Verwendung gemäß einem der vorangehenden Ansprüche, wobei der Tumor wenigstens einer ist, der ausgewählt ist aus Lungenkrebs, Kolonkrebs, Magenkrebs, Brustkrebs, Ösophaguskrebs, Kopf- und Halskrebs, Leberkrebs, Pankreaskrebs, Gallenblasen/Gallenleiterkrebs, Prostatakrebs, Blasenkrebs, Zervixkrebs, Schilddrüsenkrebs, Eierstockkrebs, Leukämie, Lymphom, Sarkom und mesenchymalem Tumor.

5. Rekombinantes Adenovirus, das ein Polynucleotid umfasst, welches einen Promotor für humane Telomerase, ein E1A-Gen, eine IRES-Sequenz und ein E1B-Gen in dieser Reihenfolge enthält, zur Verwendung in einem Verfahren zur Behandlung von Tumoren in Kombination mit einer Substanz, die eine Antitumorwirkung hat, die aus Vinorelbin, einem Topoisomerase-I-Inhibitor, INGN-201 oder einer Substanz mit antimetabolischer Aktivität ausgewählt ist.

6. Substanz, die Antitumorwirkung hat, die aus Vinorelbin, einem Topoisomerase-I-Inhibitor, INGN-201 oder einer Substanz mit antimetabolischer Aktivität ausgewählt ist, zur Verwendung in einem Verfahren zur Behandlung von Tumoren in Kombination mit einem rekombinanten Adenovirus, das ein Polynucleotid umfasst, welches einen Promotor für humane Telomerase, ein E1A-Gen, eine IRES-Sequenz und ein E1B-Gen in dieser Reihenfolge enthält.

7. Adenovirus zur Verwendung gemäß Anspruch 5 oder Substanz zur Verwendung gemäß Anspruch 6, wobei das Adenovirus ein Polynucleotid umfasst, das einen hTERT-Promotor als den Promotor enthält.

8. Adenovirus zur Verwendung gemäß Anspruch 5 oder 7 oder Substanz zur Verwendung gemäß Anspruch 6 oder 7, wobei die Substanz mit antimetabolischer Aktivität ein Folat, ein Purin, ein Pyrimidin, Fluoruracil, Tegafur oder Carmofur ist.

9. Adenovirus zur Verwendung gemäß Anspruch 5, 7 oder 8 oder Substanz zur Verwendung gemäß Anspruch 6, 7 oder 8, wobei der Tumor wenigstens einer ist, ausgewählt aus Lungenkrebs, Kolonkrebs, Magenkrebs, Brustkrebs, Ösophaguskrebs, Kopf- und Halskrebs, Leberkrebs, Pankreaskrebs, Gallenblasen/Gallenleiterkrebs, Prostatakrebs, Blasenkrebs, Zervixkrebs, Schilddrüsenkrebs, Eierstockkrebs, Leukämie, Lymphom, Sarkom und mesenchymalem Tumor.

10. Verwendung eines rekombinanten Adenovirus, das ein Polynucleotid umfasst, welches einen Promotor für humane Telomerase, ein E1A-Gen, eine IRES-Sequenz und ein E1B-Gen in dieser Reihenfolge enthält, zur Herstellung eines Medikaments zur Verwendung in einem Verfahren zur Behandlung von Tumoren in Kombination mit einer Substanz, die eine Antitumorwirkung hat, die aus Vinorelbin, einem Topoisomerase-I-Inhibitor, INGN-201 oder einer Substanz mit antimetabolischer Aktivität ausgewählt ist.

11. Verwendung einer Substanz, die Antitumorwirkung hat, die aus Vinorelbin, einem Topoisomerase-I-Inhibitor, INGN-201 oder einer Substanz mit antimetabolischer Aktivität ausgewählt ist, zur Herstellung eines Medikaments zur Verwendung in einem Verfahren zur Behandlung von Tumoren in Kombination mit einem rekombinanten Adenovirus, das ein Polynucleotid umfasst, welches einen Promotor für humane Telomerase, ein E1A-Gen, eine IRES-Sequenz und ein E1B-Gen in dieser Reihenfolge enthält.

12. Verwendung eines Adenovirus gemäß Anspruch 10 oder Verwendung einer Substanz gemäß Anspruch 11, wobei das Adenovirus ein Polynucleotid umfasst, welches einen hTERT-Promotor als den Promotor enthält.

13. Verwendung eines Adenovirus gemäß Anspruch 10 oder 12 oder Verwendung einer Substanz gemäß Anspruch 11 oder 12, wobei die Substanz mit antimetabolischer Aktivität ein Folat, ein Purin, ein Pyrimidin, Fluoruracil, Tegafur oder Carmofur ist.

## Revendications

1. Composition pharmaceutique pour utilisation dans un procédé de traitement de tumeurs par thérapie combinée, comprenant un adénovirus recombiné qui comporte un polynucléotide contenant un promoteur pour télomérase humaine, un gène E1A, une séquence IRES et un gène E1B, dans l'ordre indiqué, et une substance à effet anti-tumoral, dans laquelle la substance à effet anti-tumoral est la vinorelbine, un inhibiteur de topoisomérase I, l'INGN-201, ou une substance possédant une activité antimétabolique.

2. Composition pharmaceutique pour utilisation conforme à la revendication 1, dans laquelle l'adénovirus comporte un susdit polynucléotide qui contient un promoteur hTERT en tant que ledit promoteur.

3. Composition pharmaceutique pour utilisation conforme à la revendication 1 ou 2, dans laquelle la substance possédant une activité antimétabolique est un folate, une purine, une pyrimidine, le fluorouracile, le tegafur ou le carmofur.

4. Composition pharmaceutique pour utilisation conforme à l'une des revendications précédentes, pour laquelle la tumeur est au moins une tumeur choisie parmi les suivantes : cancer du poumon, cancer du côlon, cancer de l'estomac, cancer du sein, cancer de l'oesophage, cancer de la tête et du cou, cancer du foie, cancer du pancréas, cancer de la vésicule et des canaux biliaires, cancer de la prostate, cancer de la vessie, cancer du col de l'utérus, cancer de la thyroïde, cancer des ovaires, leucémie, lymphome, sarcome, et tumeur mésenchymateuse.

5. Adénovirus recombiné comportant un polynucléotide qui contient un promoteur pour télomérase humaine, un gène E1A, une séquence IRES et un gène E1B, dans l'ordre indiqué, pour utilisation dans un procédé de traitement de tumeurs en combinaison avec une substance à effet anti-tumoral, choisie parmi la vinorelbine, un inhibiteur de topoisomérase I, l'INGN-201 et une substance possédant une activité antimétabolique.

6. Substance à effet anti-tumoral, choisie parmi la vinorelbine, un inhibiteur de topoisomérase I, l'INGN-201 et une substance possédant une activité antimétabolique, pour utilisation dans un procédé de traitement de tumeurs en combinaison avec un adénovirus recombiné comportant un polynucléotide qui contient un promoteur pour télomérase humaine, un gène E1A, une séquence IRES et un gène E1B, dans l'ordre indiqué.

7. Adénovirus pour utilisation conforme à la revendication 5, ou substance pour utilisation conforme à la revendication 6, dans lequel ou laquelle l'adénovirus comporte un susdit polynucléotide qui contient un promoteur hTERT en tant que ledit promoteur.

8. Adénovirus pour utilisation conforme à la revendication 5 ou 7, ou substance pour utilisation conforme à la revendication 6 ou 7, dans lequel ou laquelle la substance possédant une activité antimétabolique est un folate, une purine, une pyrimidine, le fluoro-uracile, le tegafur ou le carmofur.

9. Adénovirus pour utilisation conforme à la revendication 5, 7 ou 8, ou substance pour utilisation conforme à la revendication 6, 7 ou 8, pour lequel ou laquelle la tumeur est au moins une tumeur choisie parmi les suivantes : cancer du poumon, cancer du côlon, cancer de l'estomac, cancer du sein, cancer de l'oesophage, cancer de la tête et du cou, cancer du foie, cancer du pancréas, cancer de la vésicule et des canaux biliaires, cancer de la prostate, cancer de la vessie, cancer du col de l'utérus, cancer de la thyroïde, cancer des ovaires, leucémie, lymphome, sarcome, et tumeur mésenchymateuse.

10. Utilisation d'un adénovirus recombiné comportant un polynucléotide qui contient un promoteur pour télomérase humaine, un gène E1A, une séquence IRES et un gène E1B, dans l'ordre indiqué, pour la fabrication d'un médicament destiné à être utilisé dans un procédé de traitement de tumeurs en combinaison avec une substance à effet anti-tumoral, choisie parmi la vinorelbine, un inhibiteur de topoisomérase I, l'INGN-201 et une substance possédant une activité antimétabolique.

11. Utilisation d'une substance à effet anti-tumoral, choisie parmi la vinorelbine, un inhibiteur de topoisomérase I, l'INGN-201 et une substance possédant une activité antimétabolique, pour la fabrication d'un médicament destiné à être utilisé dans un procédé de traitement de tumeurs en combinaison avec un adénovirus recombiné comportant un polynucléotide qui contient un promoteur pour télomérase humaine, un gène E1A, une séquence IRES et un gène E1B, dans l'ordre indiqué.

12. Utilisation d'un adénovirus, conforme à la revendication 10, ou utilisation d'une substance, conforme à la revendication 11, dans laquelle l'adénovirus comporte un susdit polynucléotide qui contient un promoteur hTERT en tant que ledit promoteur.

13. Utilisation d'un adénovirus, conforme à la revendication 10 ou 12, ou utilisation d'une substance, conforme à la revendication 11 ou 12, dans laquelle la substance possédant une activité antimétabolique est un folate, une purine, une pyrimidine, le fluoro-uracile, le tegafur ou le carmofur.
